# EUROPEAN PATENT APPLICATION

(11) **EP 4 234 701 A2**
(43) Date of publication of application: **30.08.2023**
(21) Application number: 23160014.9
(22) Date of filing: 31.12.2018
(51) Int. Cl.: C12N 15/82, A01H 4/00

(54) **REGENERATION OF GENETICALLY MODIFIED PLANTS**

(30) Priority: 03.01.2018 EP 18150187
(62) Divisional of application: 18827136.5
(71) Applicant: BASF SE, 67056 Ludwigshafen am Rhein (DE); KWS SAAT SE & Co. KGaA, 37574 Einbeck (DE)
(72) Inventor: PACHECO VILLALOBOS, David, 37574 Einbeck (DE); KOCH, Wolfgang, 37574 Einbeck (DE); POLLET, Bruno, 9850 Nevele (DE); SCHMITZ, Oliver, 14624 Dallgow-Döberitz (DE); KONG, Jixiang, 37574 Einbeck (DE); MARTIN-ORTIGOSA, Susana, 37574 Einbeck (DE)
(74) Representative: Heubeck, Christian

(57) **Abstract**

The present invention relates to the field of plant breeding and in particular to the generation of plants from cells and other tissues. More particularly, the invention provides methods and means for improving plant regeneration, especially from transformed or genetically modified plant cells.

## Description

The present invention relates to the field of plant breeding and biotechnology and in particular to the generation of plants from cells and other tissues. More particularly, the invention provides methods and means for improving plant regeneration, especially from transformed or genetically modified plant cells.

In plant breeding, the process of manipulation of plant species has been practiced since near the beginning of human civilization in order to create desired genotypes and phenotypes for specific purposes. With the development of genetic engineering, this field of agriculture has significantly changed during the last decades. A variety of methods for plant genetic engineering has been developed. The choice of transformation method depends on a number of variables, primarily the plant species to be transformed, the purpose of the experiment and the availability of the necessary equipment. The vast majority of plant transformation techniques requires the use of explants with high regeneration capacities as starting material. In addition, gene editing constitutes a new molecular biological method by means of which specific modifications such as insertions, deletions or point mutations or combinations thereof can be introduced into the genome of a plant. To this end, specific molecular instruments are required which firstly have nuclease activity, but above all can be guided to the target sequence to be modified with sufficient specificity to program and carry out a specific and site-directed mutagenesis. In the past few years in plant biotechnology, specific genome editing has developed into an alternative to conventional breeding and to transgenic strategies. However, tools which are currently available, such as meganucleases, zinc finger nucleases (ZFNs), "transcription activator-like effector nucleases" (TALENs) or CRISPR systems are only used in plant biotechnology to a limited extent because of limited regeneration capacities of edited starting material of plants.

A wide variety of cells have the potential to develop into embryos, including haploid gametophytic cells, such as the cells of pollen and embryo sacs (see Forster, B.P., et al. (2007) Trends Plant Sci. 12: 368-375 and Segui-Simarro, J.M. (2010) Bot. Rev. 76: 377-404), as well as somatic cells derived from all three fundamental tissue layers of the plant (Gaj, M.D. (2004) Plant Growth Regul. 43: 27-47 or Rose, R., et al. (2010) "Developmental biology of somatic embryogenesis" in: Plant Developmental Biology-Biotechnological Perspectives, Pua E-C and Davey MR, Eds. (Berlin Heidelberg: Springer), pp. 3-26).

The ability to regenerate into plants is often limited to particular genotypes in a certain plant species and weakened in transformed and genetically modified plant cells and other precursor tissues. Even if the step of transformation and genetic modification of a plant cell is successful, this does not necessarily mean that the desired plants can actually be obtained from the modified cells. It is assumed that the treatment, which the plant cells and other precursor tissues are subjected to in order to achieve a genetic modification, affects plant development and regeneration. Thus, it is an object of the present invention to improve efficacy of previously known methods for generating transgenic and genetically modified plants and to support the regeneration of plants from modified plant cells and other plant precursors.

In the present invention, it was surprisingly found that the Arabidopsis gene GRF5 (*GROWTH-REGULATING FACTOR 5*) has an effect in boosting plant regeneration that has never been reported for this gene or its counterparts of the GRF gene family.

In van der Knaap et al. (2000; "A novel gibberellin-induced gene from rice and its potential regulatory role in stem growth", Plant physiology, 122(3), 695-704.) the authors have identified and characterized the first member of the GRF gene family in rice (*OsGRF1*). This was a gibberellic acid-induced gene in intercalary meristems. Overexpression in Arabidopsis caused impaired stem growth, female sterility and reduced male fertility. Application of gibberellic acid could not recover the stem elongation defect of transformed plants, suggesting that *OsGRF1* could participate in the GA-induced stem elongation. In 2003 Kim et al. ("The AtGRF family of putative transcription factors is involved in leaf and cotyledon growth in Arabidopsis", The Plant Journal, 36(1), 94-104.) have characterized the *Arabidopsis GRF* family and determined that the genes are mainly expressed in actively growing tissues. Analysis of null mutants and transgenic plants overexpressing *AtGRF1* and *AtGRF2* indicated that some members of the GRF family are involved in the regulation of cell expansion during leaf and cotyledon growth. In addition, overexpressor plants showed delayed bolting time, revealing a putative role in flowering. In a study to determine the molecular mechanisms that coordinate cell proliferation in developing leaves, Rodriguez et al. discovered that the miR396 antagonizes the expression pattern of its targets, the GRF transcription factors, in Arabidopsis ((2010), "Control of cell proliferation in Arabidopsis thaliana by microRNA miR396", Development, 137(1), 103-112.). Thus, the balance between miR396 and the *GRFs* controls the final number of cells in leaves. Furthermore, the authors showed that miR396-targeted *GRFs* can regulate the size of the shoot apical meristem.

In 2005, Horiguchi et al. ("The transcription factor AtGRF5 and the transcription coactivator AN3 regulate cell proliferation in leaf primordia of Arabidopsis thaliana", The Plant Journal, 43(1), 68-78.) first characterized *AtGRF5* and its interacting partner *ANGUSTIFOLIA3 (AN3).* Knock-out mutants *atgrf5* and *an3* developed narrow leaves due to decreased cell number, whereas cell proliferation in leaf primordia was enhanced in *AtGRF5* and *AN3* overexpressor lines. Kuijt et al. ((2014), "Interaction between the GROWTH-REGULATING FACTOR and KNOTTED1-LIKE HOMEOBOX Families of Transcription Factors", Plant physiology, 164(4), 1952-1966.) showed that members of the *GRF* family act as players in the network controlling the expression of *KNOTTED1-LIKE HOMEBOX* (*KNOX*) genes which are involved in restriction of cell differentiation in the shoot apical meristem. *AtGRF4, AtGRF5* and *AtGRF6* are able to bind to the promoter of a *KNOX* gene, repressing its expression. Arabidopsis seedlings overexpressing *AtGRF4, AtGRF5,* or *AtGRF6* show developmental aberrations in the shoot apical meristem. In a recent study of Vercruyssen et al. ((2015), "Growth regulating factor 5 stimulates Arabidopsis chloroplast division, photosynthesis, and leaf longevity", Plant physiology, pp-114.) Arabidopsis leaves overexpressing GRF5 showed higher chloroplast number per cell, increased chlorophyll content and delayed leaf senescence.

In summary, it is well-known that the Arabidopsis gene *AtGRF5* and other *GRF* genes play a role in leaf morphogenesis and stem development. In addition, GRF genes were reported to function in flowering, seed and root development, to control the plant growth under stress conditions and to regulate the plant longevity. However, during their studies, the inventors of the present invention surprisingly found another and novel function of this gene family. *GRF5* is capable of providing a positive effect on boosting plant regeneration and thus allowing a more efficient recovery of transgenic plants. The present invention allows to improve the regeneration from diverse tissues or cells (e.g. microspores), may overcome recalcitrance to plant regeneration, in particular genotype dependency, improve the recovery of transgenic plants by e.g. co-expression of gene of interest and *GRF5,* and of genome-engineered plants by e.g. transient co-expression of genome-editing components and *GRF5,* as well as shorten the time for the production of transgenic lines and the recovery. Thus, a first aspect of the present invention is the use of *GRF5* polypeptide for improving the regenerative ability of a plant.

Any reference hereinafter to a polypeptide or protein useful in the methods of the present invention is taken to mean a *GRF5* polypeptide or *GRF5* protein as defined herein. Any reference hereinafter to a nucleic acid or polynucleotide useful in the methods of the invention (except for the nucleotide sequence of interest being transformed or the nucleic acid molecule optionally used as repair template for modifying the genome of a plant) is taken to mean a nucleic acid or polynucleotide capable of encoding such a *GRF5* polypeptide or *GRF5* protein. In one embodiment, any reference to a polypeptide/protein or nucleic acid/polynucleotide useful in the methods of the invention is to be understood to mean proteins or nucleic acids useful in the methods, constructs, expression cassettes, plant cells, plants, seeds, harvestable parts and products of the invention. The nucleic acid/polynucleotide including the mRNA(s) to be introduced into a plant cell or plant (and therefore useful in performing the methods of the invention) is any nucleic acid/polynucleotide encoding the type of polypeptide/protein which will now be described, hereafter also named "GRF5 nucleic acid", "GRF5 polynucleotide", GRF5 gene" or "GRF5 mRNA" or the like.

A "GRF5 polypeptide" or "GRF5 protein" as defined herein refers to any transcription factor preferably a 14-3-3-like protein GF14 upsilon, more preferably comprising a PFAM domain PF08880 (also known as QLQ domain) and a PFAM domain PF08879 (also known as WRC domain) when analyzed with the Interproscan software (www.ebi.ac.uk/interpro), and even more preferably comprises a PFAM domain PF08880 that finds a match of at least 90%, at least 91%, at least 92%, at least 93%, at least 94%, at least 95%, at least 96%, at least 97%, at least 98%, at least 99% or 100% coverage at or near the N-terminus of the GRF5 polypeptide and the PFAM domain PF08879 that finds a match of at least 90%, at least 91%, at least 92%, at least 93%, at least 94%, at least 95%, at least 96%, at least 97%, at least 98%, at least 99% or 100% coverage C-terminally located to the PFAM domain PF08880 of the GRF5 polypeptide, i.e. the amino acid stretch matching PF08879 is located in direction of translation behind the amino acid stretch matching PF08880. Preferably, at least one of the matches has a coverage of at least 95%, at least 96%, at least 97%, at least 98%, at least 99% or 100%.

In one embodiment, both matching amino acid stretches are located in the N-terminal half of the GRF5 polypeptide, preferably the amino acid stretch matching PFAM domain PF08880 is located in the N-terminal quarter of the *GRF5* polypeptide. Preferably, the PFAM domain PF08880 matches the amino acid residues of the *GRF5* polypeptide starting from residue 5, 6, 7, 8, 9, 10, 11, 12, 13, 14, 15, 16, 17, 18, 19, 20, 21, 22, 23, 24 or 25 of the *GRF5* polypeptide, preferably from residue 9, 10, 11, 12, 13, 14, 15, 16, 17, 18, 19, 20 or 21, and the PFAM domain PF08879 matches the amino acid residues of the *GRF5* polypeptide starting from residue 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 of the *GRF5* polypeptide, preferably from residue 72, 73, 74, 75, 76, 77, 78, 79, 80, 81, 82, 83, 84, 85, 86, 87, 88, 89, 90, 91, 92, 93, 94 or 95. Preferably, the distance between the starting amino acid residue of amino acid stretch matching PFAM domain PF08880 and the starting amino acid residue of amino acid stretch matching PFAM domain PF08879 is 60 to 82 amino acids within the *GRF5* polypeptide, more preferably 60 to 75 amino acids, more preferably 61 to 75 amino acids, even more preferably 62 to 73 amino acids within the *GRF5* polypeptide.

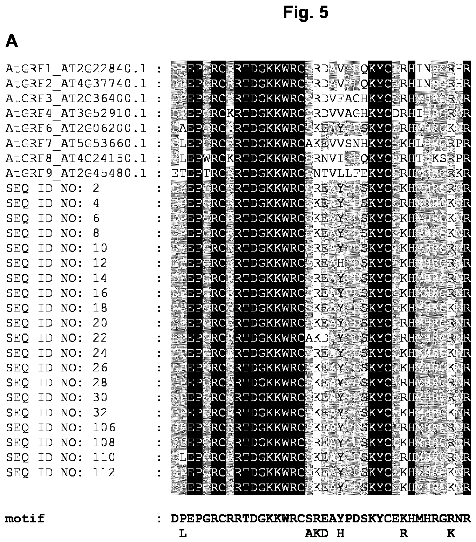

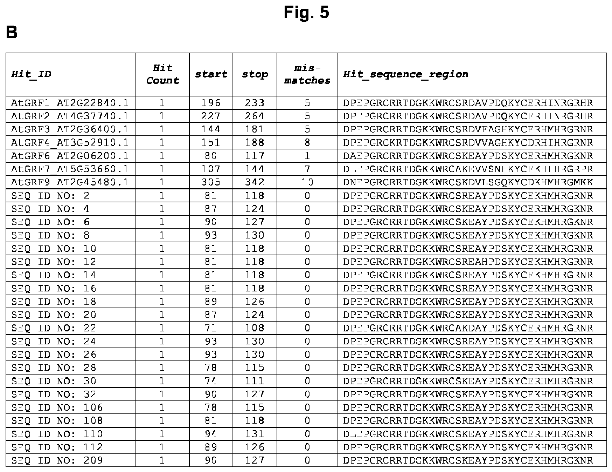

In a further embodiment, the *GRF5* polypeptide as used herein comprises the indicator motif: [D]-[PL]-[E]-[P]-[G]-[R]-[C]-[R]-[R]-[T]-[D]-[G]-[K]-[K]-[W]-[R]-[C]-[SA]-[RK]-[ED]-[A]-[YH]-[P]-[D]-[S]-[K]-[Y]-[C]-[E]-[KR]-[H]-[M]-[H]-[R]-[G]-[RK]-[N]-[R], wherein it is allowable/tolerable that the *GRF5* polypeptide exhibits a maximum number of three mismatches in comparison with the indicator motif, i.e., up to three mismatches may appear in a sequence alignment between the respective *GRF5* polypeptide and the indicator motif, preferably a maximum number of two mismatches in comparison with the indicator motif, i.e., up to two mismatches may appear in a sequence alignment between the respective *GRF5* polypeptide and the indicator motif, more preferably a maximum number of one mismatch in comparison with the indicator motif, i.e., only one mismatch may appear in a sequence alignment between the respective *GRF5* polypeptide and the indicator motif, and more preferably no mismatch in comparison with the indicator motif. In a particularly preferred embodiment, one of the mismatches or the sole mismatch is located at position 2 of the indicator motif and more preferably the mismatch is [A] instead of [P]. A mismatch means that the amino acid at a certain position according to the indicator motif is replaced by different amino acid or is deleted or shifted by the insertion of at least one additional amino acid. The letters of the indicator motif within square brackets indicate the amino acid residue (one-letter code) and the motif represents the order of the amino acid residues in direction from N-terminus to C-terminus as present in any *GRF5* polypeptide of the present invention. If there are two letters within one square bracket they represent alternatives. The motif has been deduced from a comprehensive comparison of the sequences of GRF5 polypeptides derived from 16 different plant species including monocotyledonous and dicotyledonous plants and allows to distinguish GRF5 polypeptides from other members of the GRF protein family like GRF1 (see Figure 5A). Exemplary motif analysis by sequence alignment/comparison for the determination of the number of mismatches is shown in Figure 5B. Preferably, the motif is located in the N-terminal half of the GRF5 polypeptide, more preferably the motif is located in the N-terminal half of the GRF5 polypeptide and contains a sub-region of amino acid stretch matching PFAM domain PF08879, i.e. the motif has a sequential overlap with the amino acid stretch matching PFAM domain PF08879. Preferably the indicator motif consists of any of the amino acid sequences SEQ ID NO: 41 to SEQ ID NO: 104 or SEQ ID NO: 113 to SEQ ID NO: 176. Correspondingly the *GRF5* polypeptide preferably comprises one contiguous motif consisting of any of the amino acid sequences SEQ ID NO: 41 to SEQ ID NO: 104 or SEQ ID NO: 113 to SEQ ID NO: 176.

Examples of *GRF5* polypeptide from various plant species useful in the methods of the present invention are described further below. In Table 1, the locations of the PFAM domain PF08880 and of the PFAM domain PF08879 as well as the individual coverage in any of the presented GRF5 sequences is indicated.

**Table 1: Domain analyses in GRF5 polypeptides derived from 16 different plant species**

| [SEQ ID NO] | Pfam domain (HMM) | Pfam domain description | Pfam domain length | (i)E_ value | score | Pfam domain from | Pfam domain to | sequence from | sequence to | length of Pfam domain covered % |
|---|---|---|---|---|---|---|---|---|---|---|
| 2 | PF08879 | WRC | 43 | 1.60E-20 | 72,5 | 1 | 41 | 82 | 124 | 95 |
| 2 | PF08880 | QLQ | 35 | 7,80E-16 | 57,4 | 1 | 35 | 16 | 50 | 100 |
| 4 | PF08879 | WRC | 43 | 1,10E-20 | 73 | 1 | 43 | 88 | 130 | 100 |
| 4 | PF08880 | QLQ | 35 | 3,00E-16 | 58,8 | 1 | 34 | 20 | 54 | 97 |
| 6 | PF08879 | WRC | 43 | 1,90E-20 | 72,3 | 1 | 43 | 91 | 133 | 100 |
| 6 | PF08880 | QLQ | 35 | 7,00E-14 | 51,2 | 1 | 33 | 19 | 53 | 94 |
| 8 | PF08879 | WRC | 43 | 2,00E-20 | 72,2 | 1 | 43 | 94 | 136 | 100 |
| 8 | PF08880 | QLQ | 35 | 2,20E-15 | 56 | 1 | 34 | 21 | 55 | 97 |
| 10 | PF08879 | WRC | 43 | 1,20E-20 | 72,9 | 1 | 41 | 82 | 124 | 95 |
| 10 | PF08880 | QLQ | 35 | 3,30E-16 | 58,6 | 1 | 35 | 16 | 50 | 100 |
| 12 | PF08879 | WRC | 43 | 1,80E-20 | 72,3 | 1 | 41 | 82 | 124 | 95 |
| 12 | PF08880 | QLQ | 35 | 3,30E-16 | 58,6 | 1 | 35 | 16 | 50 | 100 |
| 14 | PF08879 | WRC | 43 | 1,20E-20 | 72,9 | 1 | 41 | 82 | 124 | 95 |
| 14 | PF08880 | QLQ | 35 | 3,30E-16 | 58,6 | 1 | 35 | 16 | 50 | 100 |
| 16 | PF08879 | WRC | 43 | 1,20E-20 | 72,9 | 1 | 41 | 82 | 124 | 95 |
| 16 | PF08880 | QLQ | 35 | 4,90E-16 | 58,1 | 1 | 35 | 16 | 50 | 100 |
| 18 | PF08879 | WRC | 43 | 1,80E-20 | 72,3 | 1 | 43 | 90 | 132 | 100 |
| 18 | PF08880 | QLQ | 35 | 6,90E-14 | 51,2 | 1 | 33 | 18 | 52 | 94 |
| 20 | PF08879 | WRC | 43 | 2,50E-21 | 75,1 | 1 | 43 | 88 | 130 | 100 |
| 20 | PF08880 | QLQ | 35 | 2,30E-15 | 55,9 | 1 | 34 | 18 | 52 | 97 |
| 22 | PF08879 | WRC | 43 | 2,30E-20 | 72 | 1 | 43 | 72 | 114 | 100 |
| 22 | PF08880 | QLQ | 35 | 8,50E-16 | 57,3 | 1 | 35 | 10 | 44 | 100 |
| 24 | PF08879 | WRC | 43 | 1,10E-20 | 73 | 1 | 43 | 94 | 136 | 100 |
| 24 | PF08880 | QLQ | 35 | 2,20E-15 | 56 | 1 | 34 | 21 | 55 | 97 |
| 26 | PF08879 | WRC | 43 | 1,90E-20 | 72,2 | 1 | 43 | 94 | 136 | 100 |
| 26 | PF08880 | QLQ | 35 | 2,20E-15 | 56 | 1 | 34 | 21 | 55 | 97 |
| 28 | PF08879 | WRC | 43 | 2,80E-21 | 74,9 | 1 | 43 | 79 | 121 | 100 |
| 28 | PF08880 | QLQ | 35 | 8,50E-13 | 47,7 | 1 | 34 | 13 | 47 | 97 |
| 30 | PF08879 | WRC | 43 | 2,30E-21 | 75,2 | 1 | 43 | 75 | 117 | 100 |
| 30 | PF08880 | QLQ | 35 | 2,50E-15 | 55,8 | 2 | 34 | 9 | 43 | 94 |
| 32 | PF08879 | WRC | 43 | 1,90E-20 | 72,3 | 1 | 43 | 91 | 133 | 100 |
| 32 | PF08880 | QLQ | 35 | 3,50E-15 | 55,4 | 1 | 34 | 18 | 52 | 97 |
| 106 | PF08879 | WRC | 43 | 2,80E-21 | 74,9 | 1 | 43 | 79 | 121 | 100 |
| 106 | PF08880 | QLQ | 35 | 8,50E-13 | 47,7 | 1 | 34 | 13 | 47 | 97 |
| 108 | PF08879 | WRC | 43 | 1,20E-20 | 72,9 | 1 | 41 | 82 | 124 | 95 |
| 108 | PF08880 | QLQ | 35 | 3,30E-16 | 58,6 | 1 | 35 | 16 | 50 | 100 |
| 110 | PF08879 | WRC | - | - | - | 2 | 43 | 95 | 137 | - |
| 110 | PF08880 | QLQ | - | - | - | 1 | 37 | 13 | 49 | - |
| 112 | PF08879 | WRC | - | - | - | 1 | 43 | 90 | 131 | - |
| 112 | PF08880 | QLQ | - | - | - | 1 | 33 | 15 | 49 | - |
| 209 | PF08879 | WRC | - | - | - | 1 | 43 | 90 | 131 | - |
| 209 | PF08880 | QLQ | - | - | - | 1 | 33 | 15 | 49 | - |

According to one aspect, the invention provides a method for transforming a plant cell comprising the steps
(a1) introducing into a plant cell
   (i) at least one nucleotide sequence of interest; and
   (ii) an expression cassette comprising a polynucleotide encoding a GRF5 polypeptide, mRNA(s) encoding GRF5 polypeptide or a GRF5 polypeptide, wherein (i) and (ii) can be introduced in parallel or sequentially in any order, or
(a2) introducing into a plant cell at least one nucleotide sequence of interest; and inducing in said plant cell in parallel or sequentially an enhanced expression level of an endogenous gene encoding a GRF5 polypeptide; and
(b) optionally cultivating the plant cell of (a1) or (a2) or a plant cell derived from the plant cell of (a1) or (a2) under conditions, where in the plant cell the GRF5 polypeptide is expressed from the expression cassette, the GRF5 polypeptide is translated from introduced mRNA(s), GRF5 polypeptide(s) is enhanced/increased expressed from the endogenous gene, or the GRF5 polypeptide(s) is (are) present, preferably in an enhanced amount compared to the amount in a wild type plant cell or a plant cell into which the expression cassette comprising a polynucleotide encoding a GRF5 polypeptide, mRNA(s) encoding GRF5 polypeptide or the GRF5 polypeptide(s) has (have) not been introduced according to step (a1) or in which the enhanced expression level of an endogenous gene encoding the GRF5 polypeptide has not been induced according to step (a2).

The methods according to the present invention yields a modified or transformed plant cell having an improved ability of regeneration due to the presence of GRF5 or the presence of GRF5 in an enhanced amount. It is preferred, however, that in the modified plant cell the presence of GRF5 or the presence of GRF5 in an enhanced amount is transient.

Transformation of a plant cell means introducing a nucleic acid molecule into a plant cell in a manner to cause stable integration into the genome of the plant cell or transient appearance in the plant cell leading to expression of the nucleic acid sequence for example constitutively, temporally or specifically related to particular tissue(s) or certain developmental stage(s) et cetera. Transformation and regeneration of both monocotyledonous and dicotyledonous plant cells is now routine, and the selection of the most appropriate transformation technique will be determined by the practitioner. The choice of method will vary with the type of plant or genotype to be transformed; those skilled in the art will recognize the suitability of particular methods for given plant types or genotypes. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and Agrobacterium-mediated transformation.

Step (a1) (i) or (a2) of introducing the at least one nucleotide sequence of interest can be performed using any suitable method commonly known in the art. A number of methods is available to transfer nucleic acids of interest into plant cells. An exemplary vector mediated method is Agrobacterium-mediated transformation, as described, for example, by Lindsay & Gallois, 1990, Journal of Experimental Botany, and Kischenko et al., 2005, Cell Biology International for sugar beet, by Ishida et al., 2007, ("Agrobacterium-mediated transformation of maize." Nature protocols, 2(7), 1614-1621) for corn, or by the PureWheat Technology from Japan Tobacco company for wheat. Other suitable techniques include particle bombardment and electroporation.

The nucleotide sequence of interest according to the invention may be a DNA or RNA sequence, e.g. mRNA, siRNA, miRNA etc. More particularly, the nucleotide sequence of interest encodes at least one phenotypic trait. Preferably, the phenotypic trait conferred by the DNA or RNA can be selected from the group consisting of resistance/tolerance to biotic stress, including pathogen resistance/tolerance, wherein the pathogen can be a virus, bacterial, fungal or animal pathogen, resistance/tolerance to abiotic stress including chilling resistance/tolerance, drought stress resistance/tolerance, osmotic resistance/tolerance, heat stress resistance/tolerance, cold or frost stress resistance/tolerance, oxidative stress resistance/tolerance, heavy metal stress resistance/tolerance, salt stress or water logging resistance/tolerance, lodging resistance/tolerance, shattering resistance/tolerance, or resistance/tolerance against one or more herbicides like glyphosate, glufosinate, 2,4-D, Dicamba, ALS inhibitors et cetera. The at least one phenotypic trait of interest can also be selected from the group consisting of the modification of a further agronomic trait of interest including yield increase, flowering time modification, seed color modification, endosperm composition modification, nutritional content modification or metabolic engineering of a pathway of interest.

In context of the present invention, GRF5 can be introduced as an expression cassette comprising a polynucleotide encoding a GRF5 polypeptide, as mRNA encoding a GRF5 polypeptide (including also pre-mRNA or precursor mRNA) or as a GRF5 polypeptide. Exemplary techniques for introducing a nucleic acid molecule are described above. Alternatively, GRF5 can be provided in the plant cell by activating the expression of the endogenous gene encoding for GRF5 polypeptide. This would lead to an enhanced expression level of the endogenous *GRF5* gene, i.e. to the presence or occurrence of GRF5 polypeptide in an enhanced amount in the plant cell. The activation of the expression of the endogenous gene can be achieved by modifying the activity or structure of the promoter of the endogenous gene encoding the GRF5 polypeptide. For instances, enhancer elements can be introduced into the promoter by means of gene editing; or either an enhancer element regulating the promoter can be further strengthen or a silencer element regulating the promoter can be weakened by e.g. targeted mutagenesis/modification; or modifications can be introduced into the epigenome related to enhancers by means of gene editing tools like CRISPR systems (Hilton et al. (2015). Epigenome editing by a CRISPR-Cas9-based acetyltransferase activates genes from promoters and enhancers. Nature biotechnology, 33(5), 510-517); or synthetic transcription factors based on e.g. TALE activators or dCas9 activators can be introduced into the cell where they are able to bind targeted recognition sites on or near by the promoter und activate transcription of the GRF5 gene (Cheng et al. (2013). Multiplexed activation of endogenous genes by CRISPR-on, an RNA-guided transcriptional activator system. Cell research, 23(10), 1163.); or the amount of microRNA (miRNA) in the plant cell regulating the expression of the *GRF5* gene by post-transcriptional inhibition can be reduced by e.g. knock out (null mutant) or knock down in order to increase the amount of translated GRF5 polypeptide in the plant cell - for example Rodriguez et al. 2010 (supra) identified in Arabidopsis the microRNA miR396 which antagonizes the expression of *GRF5* and represents therefore a suitable target for affecting the amount of *GRF5* polypeptide in a plant cell.

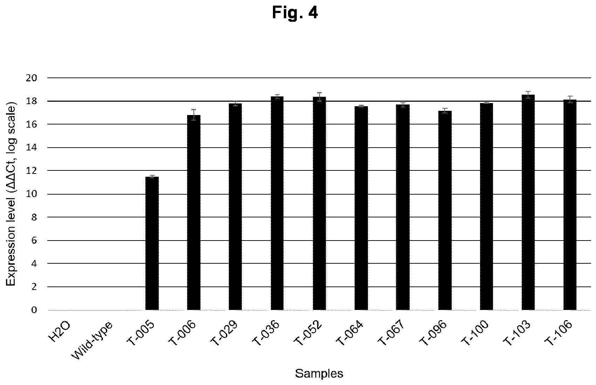

Dependent on the plant species as well as on the cell type different levels of gene or expression activation are needed in order to have adequate amount of *GRF5* polypeptide present in the plant cell at the time when regeneration takes place. There are various techniques available to a person skilled in the art in order to measure the actual expression level of an endogenous or an introduced gene, e.g., qPCR, RT-PCR, Northern blot, or microrarrays. Measurement of the expression level of the *AtGRF5* gene introduced to a *Beta vulgaris* plant is shown in Figure 4. These methods allow those skilled in the art by routine work to adjust the level of expression of the *GRF5* gene which effects improved regeneration ability from diverse tissues or somatic and reproductive cells (e.g. microspores). In a preferred embodiment, in the plant cell the expression level of an endogenous gene encoding a GRF5 polypeptide is increased at least by the factor of 2, the factor of 3, or the factor of 5, preferably by the factor of 10, the factor of 25 or factor of 50, more preferred by the factor of 100, the factor of 200, or the factor of 500.

As described further above the induction of an enhanced expression level of an endogenous gene in a plant cell can be carried out by the application of one or more activators or a precursor thereof. These can be applied to the medium in which the plant cells are cultivated and is then actively or passively absorbed by the plant cell. Furthermore, the one or more activator or a precursor thereof can be directly introduced into the plant cell by microinjection, electroporation or biolistic bombardment. Beside the above synthetic transcription activators, a number of further activators are known from the state of the art that can be used for increasing the expression level of an endogenous gene, in particular the expression level of the endogenous GRF5 gene: In the recent years, the technical fields of chemical plant genetics and chemical plant biology emerged where biological systems are treated with small molecules to specifically perturb cellular functions. Small molecules are used commercially as drugs, herbicides, and fungicides in different systems, but in recent years they are increasingly exploited also as tools for genetic regulation. For instance, chemical genetics involves the discovery of small-molecule effectors of various cellular functions through screens of compound libraries (Dejonghe & Russinova (2017). Plant Chemical Genetics: From Phenotype-Based Screens to Synthetic Biology. Plant Physiology, pp-01805; Kawasumi, M., & Nghiem, P. (2007). Chemical genetics: elucidating biological systems with small-molecule compounds. Journal of Investigative Dermatology, 127(7), 1577-1584.). Such small molecule effectors suitable for the activation of the expression of a target gene like GRF5, can be identified by chemical screens following different strategies (Dejonghe & Russinova, 2017). Comprehensive compound libraries are available which allow the simple screening of countless small molecules and the identification of effectors which can be used for activation of the gene expression of genes like *GRF5.* As mentioned above another approach to enhance the expression level of an endogenous gene like *GRF5* is the application of so-called synthetical transcription activators. They are typically designed by the fusion of a recognition domain and at least one activator domain. The recognition domain can be derived from known systems like Zinc finger, TAL effectors or CRISPR; for activation, fusing for instances the herpes simplex virus derived VP-16 or VP-64 activation domains to a recognition domain can cause an increase in transcription. Weaker activation domains such as the AD of human NF-κB add to the variety of options for gene activation. Furthermore, as shown on endogenous promoters, combinations of activators can be used to introduce synergistic effects (Moore et al. (2014). "Transcription activator-like effectors: a toolkit for synthetic biology." ACS synthetic biology, 3(10), 708-716.; US 2002/0046419 A1; Lowder et al. (2017). "Multiplexed transcriptional activation or repression in plants using CRISPR-dCas9-based systems." Plant Gene Regulatory Networks: Methods and Protocols, 167-184.). The synthetical transcription activator can be delivered to the plant cell or introduced into the plant cell also as precursor, i.e. as DNA or RNA molecule encoding such artificial or synthetical transcription activator or a domain thereof or as inactive form of transcription activator which is activated later in the cell or a in a specific compartment of the cell. Finally, enhancing expression of *GRF* genes can be also achieved by the inactivation of upstream negative regulators (i.e. miR396) or by the creation of a mutant version of the *GRF* gene that is resistant to such negative regulators.

Preferably, the GRF5 polypeptide of the present invention comprises an amino acid sequence selected from the group consisting of SEQ ID NO: 2, 4, 6, 8, 10, 12, 14, 16, 18, 20, 22, 24, 26, 28, 30, 32, 106, 108, 110, 112 or 209, or an amino acid sequence having at least 70% identity to SEQ ID NO: 2, 4, 6, 8, 10, 12, 14, 16, 18, 20, 22, 24, 26, 28, 30, 32, 106, 108, 110, 112 or 209, preferably at least 80%, at least 85%, at least 90%, more preferably at least 95%, at least 98% or at least 99% identity to SEQ ID NO: 2, 4, 6, 8, 10, 12, 14, 16, 18, 20, 22, 24, 26, 28, 30, 32, 106, 108, 110, 112 or 209, and preferably comprises an indicator motif as described herein, particularly preferably an indicator motif consisting of any of the amino acid sequences SEQ ID NO: 41 to SEQ ID NO: 104 or SEQ ID NO: 113 to SEQ ID NO: 176. The GRF5 polypeptide, for example encoded by an endogenous gene, may comprise an amino acid sequence selected from the group consisting of the sequences of SEQ ID NO: 2 (*Arabidopsis thaliana*)*,* SEQ ID NO: 4 (*Beta vulgaris*)*,* SEQ ID NO: 6 (*Zea mays*)*,* SEQ ID NO: 8 (*Triticum aestivum*)*,* SEQ ID NO: 10 *(Brassica napus),* SEQ ID NO: 12 (*Brassica rapa*)*,* SEQ ID NO: 14 (*Brassica oleracea*)*,* SEQ ID NO: 16 (*Raphanus sativus*)*,* SEQ ID NO: 18 (*Sorghum bicolor*)*,* SEQ ID NO: 20 *(Helianthus annuus),* SEQ ID NO: 22 (*Solanum tuberosum*)*,* SEQ ID NO: 24 (*Hordeum vulgare*)*,* SEQ ID NO: 26 (*Secale cereale*)*,* SEQ ID NO: 28 *(Glycine max),* SEQ ID NO: 30 (*Gossypium hirsutum*)*,* SEQ ID NO: 32 (*Oryza sativa*)*,* SEQ ID NO: 106 *(Glycine max),* SEQ ID NO: 108 *(Brassica napus),* SEQ ID NO: 110 *(Helianthus annuus),* SEQ ID NO: 112 *(Zea mays)* or SEQ ID NO: 209 *(Zea mays).*

An exogenous (heterologous) or endogenous polynucleotide encoding the GRF5 polypeptide of the invention comprises
(i) a nucleotide sequence comprising SEQ ID NO: 1, 3, 5, 7, 9, 11, 13, 15, 17, 19, 21, 23, 25, 27, 29, 31, 105, 107, 109, 111, 207, 208 or 210;
(ii) a nucleotide sequence comprising a sequence being at least 70%, preferably at least 80%, at least 85%, at least 90%, more preferably at least 95%, at least 98% or at least 99% identical to a nucleotide sequence comprising SEQ ID NO: 1, 3, 5, 7, 9, 11, 13, 15, 17, 19, 21, 23, 25, 27, 29, 31, 105, 107, 109, 111, 207, 208 or 210;
(iii) a nucleotide sequence encoding a GRF5 polypeptide as defined above or a nucleotide sequence encoding a polypeptide encoded by (i) and/or (ii) within the scope of the degeneracy of the genetic code;
(iv) a nucleotide sequence complementary to a nucleotide sequence of (i), (ii) or (iii); or/and
(v) a nucleotide sequence hybridizing with a nucleotide sequence of (iv) under stringent condition.

The polynucleotide encoding the GRF5 polypeptide, particularly the polynucleotide encoding the *GRF5* polypeptide, may comprise a nucleotide sequence selected from the group consisting of the sequences of SEQ ID NO: 1 (*Arabidopsis thaliana*); SEQ ID NO: 3 (*Beta vulgaris*)*,* SEQ ID NO: 5 *(Zea mays),* SEQ ID NO: 7 (*Triticum aestivum*)*,* SEQ ID NO: 9 *(Brassica napus),* SEQ ID NO: 11 (*Brassica rapa*)*,* SEQ ID NO: 13 (*Brassica oleracea*)*,* SEQ ID NO: 15 (*Raphanus sativus*)*,* SEQ ID NO: 17 (*Sorghum bicolor*)*,* SEQ ID NO: 19 *(Helianthus annuus),* SEQ ID NO: 21 (*Solanum tuberosum*)*,* SEQ ID NO: 23 (*Hordeum vulgare*)*,* SEQ ID NO: 25 (*Secale cereale*)*,* SEQ ID NO: 27 *(Glycine max),* SEQ ID NO: 29 (*Gossypium hirsutum*)*,* SEQ ID NO: 31 (*Oryza sativa*)*,* SEQ ID NO: 105 *(Glycine max),* SEQ ID NO: 107 *(Brassica napus),* SEQ ID NO: 109 *(Helianthus annuus),* SEQ ID NO: 111 *(Zea mays),* SEQ ID NO: 207 (synthetic), SEQ ID NO: 208 (synthetic) or SEQ ID NO: 210 (synthetic).

For the purpose of this invention, the *"sequence identity"* of two related nucleotide or amino acid sequences, expressed as a percentage, refers to the number of positions in the two optimally aligned sequences which have identical residues (x100) divided by the number of positions compared. A gap, i.e. a position in an alignment where a residue is present in one sequence but not in the other, is regarded as a position with non-identical residues. The alignment of the two sequences is performed by the Needleman and Wunsch algorithm (Needleman and Wunsch 1970). The computer-assisted sequence alignment above, can be conveniently performed using standard software program such as program NEEDLE as implemented in the The European Molecular Biology Open Software Suite (EMBOSS), e.g. version 6.3.1.2 (Trends in Genetics 16 (6), 276 (2000)), with its default parameter, e.g. for proteins matrix = EBLOSUM62, gapopen = 10.0 and gapextend = 0.5.

The terms *"stringent conditions"* or *"hybridization under stringent conditions"* refer to conditions under which nucleotide sequences with sufficient complementarity to one another usually remain hybridized. These stringent conditions are known to the skilled person and described, for example, in Current Protocols in Molecular Biology, John Wiley & Sons, N.Y. (1989) 6.3.1-6.3.6. The skilled person knows how to determine the required hybridization conditions on the basis of, for example, Sambrook et al., Molecular Cloning, Cold Spring Harbour Laboratory, 1989. The term *"hybridization conditions"* in this respect refers not only to the actual conditions prevailing during actual agglomeration of the nucleic acids, but also to the conditions prevailing during the subsequent washing steps. Examples of stringent hybridization conditions are conditions under which primarily only those nucleic acid molecules that have at least at least 80%, preferably at least 85%, at least 90%, at least 95%, at least 98% or at least 99% sequence identity undergo hybridization. Stringent hybridization conditions are, for example: 4×SSC at 65°C and subsequent multiple washes in 0.1×SSC at 65°C. for approximately 1 hour. The term "stringent hybridization conditions" as used herein may also mean: hybridization at 68°C in 0.25 M sodium phosphate, pH 7.2, 7% SDS, 1 mM EDTA and 1% BSA for 16 hours and subsequently washing twice with 2×SSC and 0.1% SDS at 68°C. Preferably, hybridization takes place under stringent conditions.

The expression *"operably linked"* means that said elements of the chimeric gene are linked to one another in such a way that their function is coordinated and allows expression of the coding sequence, i.e. they are functionally linked. By way of example, a promoter is functionally linked to another nucleotide sequence when it is capable of ensuring transcription and ultimately expression of said other nucleotide sequence. Two proteins encoding nucleotide sequences are functionally or operably linked to each other if they are connected in such a way that a fusion protein of first and second protein or polypeptide can be formed.

A gene is said to be expressed when it leads to the formation of an expression product. An expression product denotes an intermediate or end product arising from the transcription and optionally translation of the nucleic acid, DNA or RNA, coding for such product, e. g. the second nucleic acid described herein. During the transcription process, a DNA sequence under control of regulatory regions, particularly the promoter, is transcribed into an RNA molecule. An RNA molecule may either itself form an expression product or be an intermediate product when it is capable of being translated into a peptide or protein. A gene is said to encode an RNA molecule as expression product when the RNA as the end product of the expression of the gene is, e.g., capable of interacting with another nucleic acid or protein. Examples of RNA expression products include inhibitory RNA such as e.g. sense RNA (co-suppression), antisense RNA, ribozymes, miRNA or siRNA, mRNA, rRNA and tRNA. A gene is said to encode a protein as expression product when the end product of the expression of the gene is a protein or peptide.

A nucleic acid (molecule) or nucleotide (sequence) or polynucleotide, as used herein, refers to both DNA and RNA. DNA also includes cDNA and genomic DNA. A nucleic acid molecule can be single- or double-stranded, and can be synthesized chemically or produced by biological expression *in vitro* or even *in vivo.*

It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

As used herein *"comprising"* or the like is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined may comprise additional DNA regions etc.

By means of GRF5, a plant cell or other plant precursor tissues can be provided having an improved ability of regeneration. This is particularly helpful for genetically modified plant cells, in particular plant cells with an edited genome.

According to a preferred embodiment of the invention, step (a1) of introducing the at least one nucleotide sequence of interest and GRF5 or the step (a2) of introducing the at least one nucleotide sequence of interest and inducing an enhanced expression level of an endogenous gene encoding the GRF5 yields in transient transformation of the plant cell. In terms of the invention, *"transient transformation"* means that the inserted sequence is not (stably) integrated into the genome of the plant cell. In another embodiment, a stable transformation is effected, wherein the nucleotide sequence of interest in step (a1) and (a2) of the method for transforming disclosed here and/or the polynucleotide encoding an GRF5 polypeptide in step (a1) of the method of modifying the genome disclosed here is inserted into the genome of the plant cell. According to an especially preferred embodiment of the invention, the nucleotide sequence encoding GRF5 is transformed transiently into the cell while the nucleotide sequence of interest is stably transformed into the genome of the cell.

Modifying the genome of the plant cell can be accomplished by means of a double-stranded DNA break (DSB) inducing enzyme which preferably recognizes a predetermined site in the genome of said cell.

Thus, another embodiment of the present invention is a method for modifying the genome of a plant cell comprising the steps
(a1) introducing into a plant cell an expression cassette comprising a polynucleotide encoding a GRF5 polypeptide, mRNA(s) encoding GRF5 polypeptide (including pre-mRNA(s)), or GRF5 polypeptide(s); or
(a2) inducing in a plant cell an enhanced expression level of an endogenous gene encoding a GRF5 polypeptide; and
(b) cultivating the plant cell of (a1) or (a2) or a plant cell derived from the plant cell of (a1) or (a2) under conditions where in the plant cell the GRF5 polypeptide is expressed from the expression cassette, GRF5 polypeptide is translated from introduced mRNA(s), GRF5 polypeptide is enhanced/increased expressed from the endogenous gene, or GRF5 polypeptide(s) are present, preferably in an enhanced amount compared to the amount in a wild type plant cell or a plant cell into which the expression cassette comprising a polynucleotide encoding a GRF5 polypeptide, mRNA(s) encoding GRF5 polypeptide or the GRF5 polypeptide has not been introduced according to step (a1) or in which the enhanced expression level of an endogenous gene encoding a GRF5 polypeptide has not been induced according to step (a2);
(c) modifying the genome of the plant cell of (b) by means of a double-stranded DNA break (DSB) inducing enzyme which preferably recognizes a predetermined site in the genome of said cell, and optionally by means of a repair nucleic acid molecule,
wherein the modification of said genome at said predetermined site is selected from
   i. a replacement of at least one nucleotide;
   ii. a deletion of at least one nucleotide;
   iii. an insertion of at least one nucleotide; or
   iv. any combination of i. - iii.; and
wherein step (c) is conducted simultaneously with step (a1)/(a2) and/or (b), before step (a1)/(a2), between step (a1)/(a2) or (b) or after step (b).

As used herein, a *"double-stranded DNA break inducing enzyme"* or *"DSBI enzyme"* is an enzyme capable of inducing a double-stranded DNA break at a particular nucleotide sequence, called the *"recognition site".* The double-stranded DNA break (DSB)-inducing enzyme can, for example, be selected from the group consisting of meganuclease, TAL effector nuclease, zinc finger nuclease, CRISPR systems like CRISPRlCas9, CRISPR/Cpf1, CRISPR/CasX, CRISPR/CasY, CRISPR/Csm1 or CRISPR/MAD7. Rare-cleaving endonucleases are DSBI enzymes that have a recognition site of preferably about 14 to 70 consecutive nucleotides, and therefore have a very low frequency of cleaving, even in larger genomes such as most plant genomes. Homing endonucleases, also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double strand break in the intronless or inteinless alleles, these nucleases create recombinogenic ends, which engage in a gene conversion process that duplicates the coding sequence and leads to the insertion of an intron or an intervening sequence at the DNA level. A list of other rare cleaving meganucleases and their respective recognition sites is provided in Table I of WO 03/004659 (pages 17 to 20) (incorporated herein by reference).

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the nonspecific DNA-cleavage domain from a natural restriction enzyme, such as Fokl. Such methods have been described e.g. in WO 03/080809, WO 94/18313 or WO 95/09233 and in Isalan et al. (2001). A rapid, generally applicable method to engineer zinc fingers illustrated by targeting the HIV-1 promoter. Nature biotechnology, 19(7), 656; Liu et al. (1997). Design of polydactyl zinc-finger proteins for unique addressing within complex genomes. Proceedings of the National Academy of Sciences, 94(11), 5525-5530.).

Another example of custom-designed endonucleases includes the TALE nucleases (TALENs), which are based on transcription activator-like effectors (TALEs) from the bacterial genus *Xanthomonas* fused to the catalytic domain of a nuclease (e.g. *Fokl* or a variant thereof). The DNA binding specificity of these TALEs is defined by repeat-variable di-residues (RVDs) of tandem-arranged 34/35-amino acid repeat units, such that one RVD specifically recognizes one nucleotide in the target DNA. The repeat units can be assembled to recognize basically any target sequences and fused to a catalytic domain of a nuclease create sequence specific endonucleases (see e.g. Boch et al. (2009). Breaking the code of DNA binding specificity of TAL-type III effectors. Science, 326(5959), 1509-1512; Moscou & Bogdanove (2009). A simple cipher governs DNA recognition by TAL effectors. Science, 326(5959), 1501-1501; and WO 2010/079430, WO 2011/072246, WO 2011/154393, WO 2011/146121, WO 2012/001527, WO 2012/093833, WO 2012/104729, WO 2012/138927, WO 2012/138939). WO 2012/138927 further describes monomeric (compact) TALENs and TALEs with various catalytic domains and combinations thereof.

Recently, a new type of customizable endonuclease system has been described; the so-called CRISPR/Cas system. A CRISPR system in its natural environment describes a molecular complex comprising at least one small and individual non-coding RNA in combination with a Cas nuclease or another CRISPR nuclease like a Cpf1 nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into 2 classes comprising five types of CRISPR systems, the type II system, for instance, using Cas9 as effector and the type V system using Cpf1 as effector molecule (Makarova et al., Nature Rev. Microbiol., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova *et al.,* 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which system contains both a crRNA and also a trans-activating RNA (tracrRNA) as the guide motif. These hybridize and form double-stranded (ds) RNA regions which are recognized by RNAselll and can be cleaved in order to form mature crRNAs. These then in turn associate with the Cas molecule in order to direct the nuclease specifically to the target nucleic acid region. Recombinant gRNA molecules can comprise both the variable DNA recognition region and also the Cas interaction region and thus can be specifically designed, independently of the specific target nucleic acid and the desired Cas nuclease. As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpf1 nucleases it has been described that the Cpf1-crRNA complex, without a tracrRNA, efficiently recognize and cleave target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized. Further CRISPR effectors like CasX and CasY effectors originally described for bacteria, are meanwhile available and represent further effectors, which can be used for genome engineering purposes (Burstein et al., "New CRISPR-Cas systems from uncultivated microbes", Nature, 2017, 542, 237-241).

The cleavage site of a DSBI enzyme relates to the exact location on the DNA where the double-stranded DNA break is induced. The cleavage site may or may not be comprised in (overlap with) the recognition site of the DSBI enzyme and hence it is said that the cleavage site of a DSBI enzyme is located at or near its recognition site. The recognition site of a DSBI enzyme, also sometimes referred to as binding site, is the nucleotide sequence that is (specifically) recognized by the DSBI enzyme and determines its binding specificity. For example, a TALEN or ZNF monomer has a recognition site that is determined by their RVD repeats or ZF repeats respectively, whereas its cleavage site is determined by its nuclease domain (e.g. Fokl) and is usually located outside the recognition site. In case of dimeric TALENs or ZFNs, the cleavage site is located between the two recognition/binding sites of the respective monomers, this intervening DNA region where cleavage occurs being referred to as the spacer region.

A person skilled in the art would be able to either choose a DSBI enzyme recognizing a certain recognition site and inducing a DSB at a cleavage site at or in the vicinity of the preselected/predetermined site or engineer such a DSBI enzyme. Alternatively, a DSBI enzyme recognition site may be introduced into the target genome using any conventional transformation method or by crossing with an organism having a DSBI enzyme recognition site in its genome, and any desired DNA may afterwards be introduced at or in the vicinity of the cleavage site of that DSBI enzyme.

In a particularly preferred aspect of this embodiment, a repair nucleic acid molecule is additionally introduced into the plant cell. As used herein, a *"repair nucleic acid molecule"* is a single-stranded or double-stranded DNA molecule or RNA molecule that is used as a template for modification of the genomic DNA at the preselected site in the vicinity of or at the cleavage site. As used herein, *"use as a template for modification of the genomic DNA",* means that the repair nucleic acid molecule is copied or integrated at the preselected site by homologous recombination between the flanking region(s) and the corresponding homology region(s) in the target genome flanking the preselected site, optionally in combination with non-homologous end-joining (NHEJ) at one of the two end of the repair nucleic acid molecule (e.g. in case there is only one flanking region). Integration by homologous recombination will allow precise joining of the repair nucleic acid molecule to the target genome up to the nucleotide level, while NHEJ may result in small insertions/deletions at the junction between the repair nucleic acid molecule and genomic DNA.

As used herein, "*a modification of the genome",* means that the genome has changed by at least one nucleotide. This can occur by replacement of at least one nucleotide and/or a deletion of at least one nucleotide and/or an insertion of at least one nucleotide, as long as it results in a total change of at least one nucleotide compared to the nucleotide sequence of the preselected genomic target site before modification, thereby allowing the identification of the modification, e.g. by techniques such as sequencing or PCR analysis and the like, of which the skilled person will be well aware.

As used herein "*a preselected site", "a predetermined site"* or *"predefined site"* indicates a particular nucleotide sequence in the genome (e.g. the nuclear genome or the chloroplast genome) at which location it is desired to insert, replace and/or delete one or more nucleotides. This can e.g. be an endogenous locus or a particular nucleotide sequence in or linked to a previously introduced foreign DNA or transgene. The preselected site can be a particular nucleotide position at(after) which it is intended to make an insertion of one or more nucleotides. The preselected site can also comprise a sequence of one or more nucleotides which are to be exchanged (replaced) or deleted.

As used in the context of the present application, the term "*about*" means +/- 10% of the recited value, preferably +/- 5% of the recited value. For example, about 100 nucleotides (nt) shall be understood as a value between 90 and 110 nt, preferably between 95 and 105.

As used herein, a *"flanking region",* is a region of the repair nucleic acid molecule having a nucleotide sequence which is homologous to the nucleotide sequence of the DNA region flanking (i.e. upstream or downstream) of the preselected site. It will be clear that the length and percentage sequence identity of the flanking regions should be chosen such as to enable homologous recombination between said flanking regions and their corresponding DNA region upstream or downstream of the preselected site. The DNA region or regions flanking the preselected site having homology to the flanking DNA region or regions of the repair nucleic acid molecule are also referred to as the homology region or regions in the genomic DNA.

To have sufficient homology for recombination, the flanking DNA regions of the repair nucleic acid molecule may vary in length, and should be at least about 10 nt, about 15 nt, about 20 nt, about 25 nt, about 30 nt, about 40 nt or about 50 nt in length. However, the flanking region may be as long as is practically possible (e.g. up to about 100-150 kb such as complete bacterial artificial chromosomes (BACs). Preferably, the flanking region will be about 50 nt to about 2000 nt, e.g. about 100 nt, 200 nt, 500 nt or 1000 nt. Moreover, the regions flanking the DNA of interest need not be identical to the homology regions (the DNA regions flanking the preselected site) and may have between about 80% to about 100% sequence identity, preferably about 95% to about 100% sequence identity with the DNA regions flanking the preselected site. The longer the flanking region, the less stringent the requirement for homology. Furthermore, to achieve exchange of the target DNA sequence at the preselected site without changing the DNA sequence of the adjacent DNA sequences, the flanking DNA sequences should preferably be identical to the upstream and downstream DNA regions flanking the preselected site.

As used herein, *"upstream"* indicates a location on a nucleic acid molecule which is nearer to the 5' end of said nucleic acid molecule. Likewise, the term *"downstream"* refers to a location on a nucleic acid molecule which is nearer to the 3' end of said nucleic acid molecule. For avoidance of doubt, nucleic acid molecules and their sequences are typically represented in their 5' to 3' direction (left to right).

In order to target sequence modification at the preselected site, the flanking regions must be chosen so that 3' end of the upstream flanking region and/or the 5' end of the downstream flanking region align(s) with the ends of the predefined site. As such, the 3' end of the upstream flanking region determines the 5' end of the predefined site, while the 5' end of the downstream flanking region determines the 3' end of the predefined site.

As used herein, said preselected site being located outside or away from said cleavage (and/or recognition) site, means that the site at which it is intended to make the genomic modification (the preselected site) does not comprise the cleavage site and/or recognition site of the DSBI enzyme, i.e. the preselected site does not overlap with the cleavage (and/or recognition) site. Outside/away from in this respect thus means upstream or downstream of the cleavage (and/or recognition) site.

The modified plant cell that has been transformed or gene edited according to the methods of the present invention and possibly has a modified genome can be regenerated into a whole (fertile) plant. Due to the presence of the additional GRF5 in the plant cell their ability to regenerate is significantly improved. Thus, in a preferred aspect of the invention, the transformation of a plant cell or the modification of a genome of a plant cell, respectively, is followed by a step of regenerating a plant. Accordingly, the present invention provides a method for producing a transgenic plant comprising
(a) transforming a plant cell as described hereinabove and
(b) regenerating from the plant cell of (a) or from a plant cell derived from the plant cell of (a) a plant comprising at least one plant cell which comprises the at least one nucleotide sequence of interest as a transgene.

Further, the present invention also provides a method of producing a genetically modified plant comprising
(a) modifying the genome of a plant cell as described hereinabove and
(b) regenerating from the plant cell of (a) or from a plant cell derived from the plant cell of (a) a plant comprising in at least one cell the modification of the genome or the modified plant cell.

Regeneration techniques rely on manipulation of certain phytohormones in a tissue culture growth medium, occasionally relying on a biocide and/or herbicide marker that can been introduced together with the desired nucleotide sequence(s) of interest. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, protoplasts, immature or mature embryos, embryonic tissue, meristematic tissues, organs, or parts thereof. Such regeneration techniques are described generally in Klee (1987) Ann. Rev. of Plant Phys. 38:467486. To obtain whole plants from transgenic tissues such as immature embryos, they can be grown under controlled environmental conditions in a series of media containing nutrients and hormones, a process known as tissue culture. Once whole plants are generated and produce seed, evaluation of the progeny begins.

According to the present invention, it is not only possible to improve the regeneration ability of transformed or genetically modified plant cells, but also other types of sensitive cells with poor regeneration abilities. In particular, the production of a haploid plant embryo from precursors like an immature male gametophyte or a microspore can be improved by means of *GRF5.*

Thus, another aspect of the present invention is a method of producing a haploid plant embryo comprising the steps
(a1) introducing into an immature male gametophyte or a microspore an expression cassette comprising a polynucleotide encoding a GRF5 polypeptide, mRNA(s) encoding GRF5 polypeptide, or GRF5 polypeptide(s); or
(a2) inducing in an immature male gametophyte or a microspore an enhanced expression level of an endogenous gene encoding a GRF5 polypeptide; and
(b) cultivating the immature male gametophyte or the microspore of (a) under conditions where in the immature male gametophyte or the microspore the GRF5 polypeptide is expressed from the expression cassette, GRF5 polypeptide is translated from introduced mRNA(s), GRF5 polypeptide is enhanced expressed from the endogenous gene, or GRF5 polypeptide(s) are present, preferably in an enhanced amount compared to the amount in a wild type plant cell or a plant cell into which the expression cassette comprising a polynucleotide encoding a GRF5 polypeptide, mRNA(s) encoding GRF5 polypeptide or the GRF5 polypeptide has not been introduced according to step (a1) or in which the enhanced expression level of an endogenous gene encoding a GRF5 polypeptide has not been induced according to step (a2); and
(c) selecting a haploid plant embryo derived from the immature male gametophyte or the microspore of step (b).

The invention also includes a method of producing haploid seedlings comprising exposing haploid plant material to an expression cassette comprising a polynucleotide encoding a GRF5 polypeptide, mRNA encoding GRF5 polypeptide, or *GRF5* polypeptide(s) to produce haploid embryos and then converting (i.e. germinating) the haploid embryos into seedlings. The invention therefore includes a method of making haploid plants comprising growing a seedling produced in accordance with the aforementioned method. The invention also provides a method of producing a double haploid plant comprising culturing haploid plant material in the presence of *GRF5* for a period, stimulating or allowing a spontaneous chromosome doubling, and growing the double haploid plant material into a seedling, plantlet or plant. In certain embodiments, haploid embryogenesis and chromosome doubling may take place substantially simultaneously. In other embodiments, there may be a time delay between haploid embryogenesis and chromosome doubling. Should growth of haploid seedlings, plants or plantlets not involve a spontaneous chromosome doubling event, then a chemical chromosome doubling agent may be used, e.g. colchicine. Many procedures involve contact of plant cells with colchicine, anti-microtubule agents or anti-microtubule herbicides such as pronamide, nitrous oxide, or any mitotic inhibitor. The result is homozygous doubled haploid cells. Where colchicine is used, the concentration in the medium may be generally 0.01 %-0.2%. The range of colchicine concentration may be from about 400 - 600 mg/L.

Where a microspore is exposed to *GRF5* polypeptide(s), then a callus may form and this may undergo organogenesis to form an embryo. The invention therefore includes a method of producing haploid plant callus comprising exposing an immature male gametophyte or a microspore to *GRF5* polypeptide(s) or introducing into it an expression cassette comprising a polynucleotide encoding a GRF5 polypeptide, mRNA encoding *GRF5* polypeptide, or *GRF5* polypeptide(s).

The exposure of the immature male gametophyte or the microspore to *GRF5* during cultivation step is preferably carried out for a period of time sufficient to induce haploid embryo formation. The period of time needed may depend on the species of plant concerned and these are all readily ascertainable by a person of ordinary skilled in the art. A preferred range of *GRF5* exposure is from about 1 to about 20 hours; more preferably from about 2 to about 20 hours.

In certain aspects of the invention, a physical stress is applied to the haploid plant material prior to the introduction of the expression cassette comprising a polynucleotide encoding a *GRF5* polypeptide, mRNA encoding *GRF5* polypeptide, or *GRF5* polypeptide(s). The physical stress may be any of temperature, darkness, light or ionizing radiation, starvation or osmotic stress, for example. The light may be full spectrum sunlight, or one or more frequencies selected from the visible, infrared or UV spectrum. The stresses may be continuous or interrupted (periodic); regular or random over time.

The present invention is applicable to any plant species, whether monocot or dicot. Preferably, plants which may be subject to the methods and uses of the present invention are plants of the genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus.* More preferably, the plant is selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.* Particularly preferred are *Beta vulgaris, Zea mays, Triticum aestivum, Hordeum vulgare, Secale cereale, Helianthus annuus, Solanum tuberosum, Sorghum bicolor, Brassica rapa, Brassica napus, Brassica juncacea, Brassica oleracea, Raphanus sativus, Oryza sativa, Glycine max,* and/or *Gossypium sp.*

Suitable plant cells according the present invention are especially cells of a callus tissue, preferably of a friable callus, of a meristematic tissue, of a reproductive tissue (e.g. microspores) or an embryonic tissue as well as protoplasts.

A part or parts of plants may be attached to or separated from a whole intact plant. Such parts of a plant include, but are not limited to, organs, tissues, and cells of a plant, and preferably seeds.

Subject matter of the present invention also are the plants that are obtained or obtainable by the methods described above. Accordingly, one embodiment of the invention is a transgenic plant obtained or obtainable by the above method of transforming a plant cell and regenerating a plant from said cell, as well as progeny or parts thereof, wherein the progeny or the part comprises the at least one nucleotide sequence of interest as transgene. Another embodiment of the invention is a genetically modified plant obtained or obtainable by the above method of modifying the genome of a plant cell and regenerating a plant from said cell as well as progeny or parts thereof, wherein the progeny or the part comprises the modification in the genome introduced by the above method of modification.

Further subject matter of the present invention is a plant cell or a seed derived from the above transgenic plant or genetically modified plant. Such a plant cell preferably comprises a polynucleotide encoding a GRF5 polypeptide transiently or stably integrated and a double-stranded DNA break (DSB)-inducing enzyme, which preferably recognizes a predetermined site in the genome of said cell and optionally a repair nucleic acid molecule. The polynucleotide encoding the GRF5 polypeptide is preferably operably linked to a suitable regulatory sequence so that the plant cell is capable of expressing the GRF5 polypeptide. A regulatory sequence means, for example, a "promoter" which refers to a nucleotide sequence, usually upstream (5') to its coding sequence, which controls the expression of the coding sequence by providing the recognition for RNA polymerase and other factors required for proper transcription. "Constitutive promoter" refers to promoters that direct gene expression in nearly all tissues and at all times. Examples of constitutive promoters include CaMV 35S promoter, double CaMV 35S promoter (70S promoter), nopaline synthase (nos) promoter, *BdEF1* promoter, or ubiquitin promoter like *PcUbi4* or *ZmUbi1.* "Regulated promoter" refers to promoters that direct gene expression not constitutively but in a temporally and/or spatially regulated manner and include both tissue-specific and inducible promoters. It includes natural and synthetic sequences as well as sequences which may be a combination of synthetic and natural sequences. Different promoters may direct the expression of a gene in different tissues or cell types, or at different stages of development, or in response to different environmental conditions. New promoters of various types useful in plant cells are constantly being discovered and are well-known to a person skilled in the art. "Tissue-specific promoter" refers to regulated promoters that are not expressed in all plant cells but only in one or more cell types in specific organs (such as leaves or seeds), specific tissues (such as embryo or cotyledon), or specific cell types (such as leaf parenchyma or seed storage cells). These also include promoters that are temporally regulated (such as in early or late embryogenesis), during fruit ripening in developing seeds or fruit, in fully differentiated leaf, or at the onset of senescence. "Inducible promoter" refers to those regulated promoters that can be turned on in one or more cell types by an external stimulus (such as a chemical, light, hormone, stress, or pathogen). Examples for inducible promoter are promoters inducible by ecdysone, dexamethasone, ethanol. Such promoters are well-known from the state of the art (e.g., Samalova et al. (2005). pOp6/LhGR: a stringently regulated and highly responsive dexamethasone-inducible gene expression system for tobacco. The Plant Journal, 41(6), 919-935; Gatz & Lenk (1998). Promoters that respond to chemical inducers. Trends in Plant Science, 3(9), 352-358.).

A further subject matter of the invention is a haploid plant embryo obtained or obtainable by the method of the invention.

Another subject-matter of the present invention is a plant cell comprising a polynucleotide encoding a *GRF5* polypeptide transiently or stable integrated, and a double stranded DNA break (DSB) inducing enzyme which preferably recognize a predetermined site in the genome of said cell, and optionally a repair nucleic acid molecule, wherein preferably the polynucleotide encoding the *GRF5* polypeptide being operatively linked to a suitable regulatory sequence, so that the plant cell is capable of expressing the *GRF5* polypeptide. Such plant cell can be obtained when conducting the above described method for modifying the genome of a plant cell.

A further aspect of the present invention is the use of a polynucleotide encoding a *GRF5* polypeptide, mRNA encoding a *GRF5* polypeptide, *GRF5* polypeptide or an activator of expression of an endogenous gene encoding a *GRF5* polypeptide in a method of transformation of a plant cell, preferably in the method of transformation as described above, in a method of modifying the genome of a plant cell, preferably in the method of modifying the genome as described above, in a method for the production of a plant or a haploid plant embryo, preferably in the method for the production of a plant or a haploid plant embryo as described above, or in a method for regeneration of a plant, preferably in the method for regeneration of a plant as described above.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Cray, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

All patents, patent applications, and publications or public disclosures (including publications on internet) referred to or cited herein are incorporated by reference in their entirety.

The invention will be further described with reference to the following Figures and Examples described herein. However, it is to be understood that the invention is not limited to such Examples.

### Figures

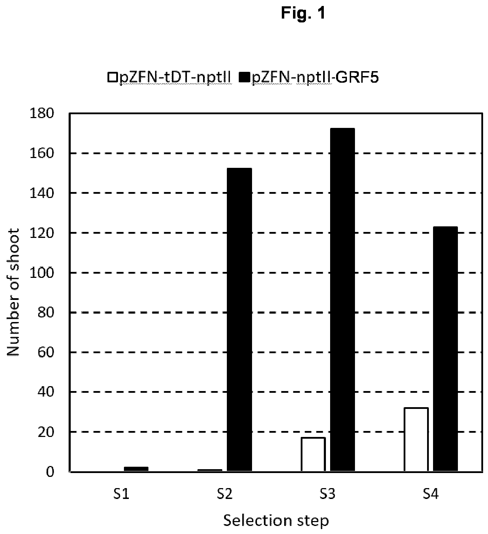

**Fig. 1****:** Total number of developing shoots in each selection step (S1-S4). Data obtained from 5 independent transformation experiments performed either with the control construct pZFN-tdT-nptll or with pZFN-nptll-GRF5 (compare table 3).

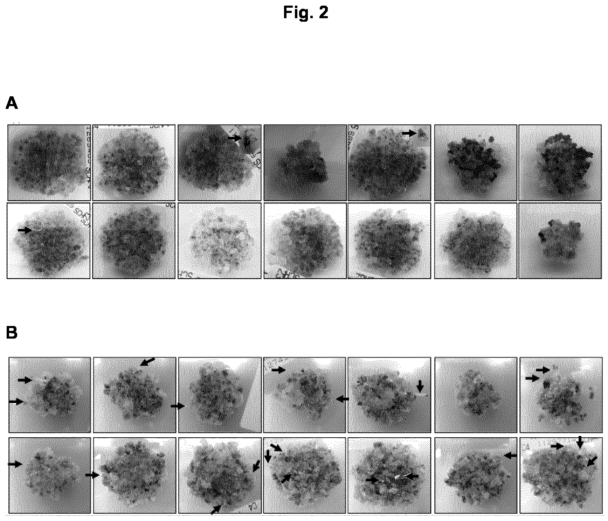

**Fig. 2****:** Shoot regeneration at the beginning of the selection step 3 (S3) of 14 independently transformed calli on a control experiment (A) and an experiment done with pZFN-nptll-GRF5 (B). Arrows indicate developing shoots. Experiments were performed following the method of 2.1.

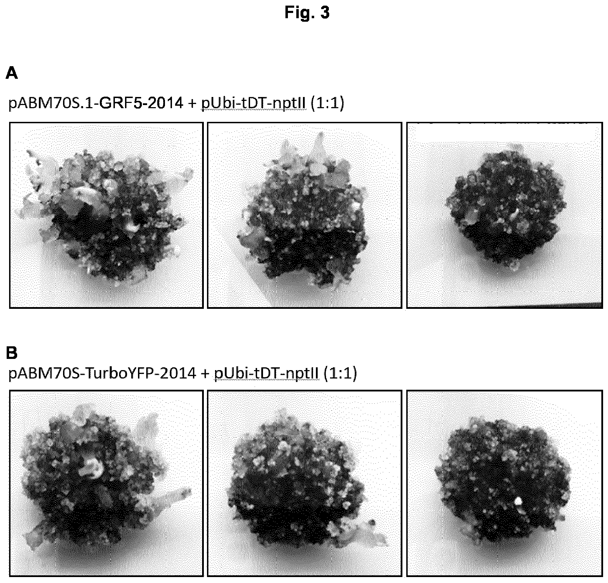

**Fig. 3****:** Shoot regeneration in sugar beet calli 10 days after bombarded with pZFN-nptll-GRF5 (A) and the control plasmid pABM70S-TurboYFP (B). The calli were co-bombarded with pUbi4-tDT plasmid to control the transient expression levels by red fluorescence. Experiments were performed following the method 2.2. Three independently bombarded calli are shown.
**Fig. 4****:** Expression level of GRF5 in eleven independent sugar beet transgenic events.
**Fig. 5****: A**: Sequence comparison of the sequences of GRF5 polypeptides derived from 16 different plant species and deduction of a GRF5 specific indicator motif (SEQ ID NO: 177); partial sequences of the sequences of GRF5 polypeptides as shown are set forth in SEQ ID NOs: 178 - 206; **B:** motif analysis by sequence alignment/comparison on the complete protein sequences derived from 16 different plant species with allowing up to 10 mismatches. The columns *"start"* and *"stop"* indicate the starting and ending position of sequence fragment of the GRF5 polypeptides corresponding to the indicator motif; column *"mismatches"* shows the number of mismatches between the respective GRF5 polypeptide and the indicator motif; partial sequences of the sequences of GRF5 polypeptides as shown are set forth in SEQ ID NOs: 178 - 184 and SEQ ID NOs: 186 - 206. AtGRF1_AT2G22840.1 (SEQ ID NO: 33); AtGRF2_AT4G37740.1 (SEQ ID NO: 34); AtGRF3_AT2G36400.1 (SEQ ID NO: 35); AtGRF4_AT3G52910.1 (SEQ ID NO: 36); AtGRF6_AT2G06200.1 (SEQ ID NO: 37); AtGRF7_AT5G53660.1 (SEQ ID NO: 38); AtGRF8_AT4G24150.1 (SEQ ID NO: 39); AtGRF9_AT2G45480.1 (SEQ ID NO: 40).

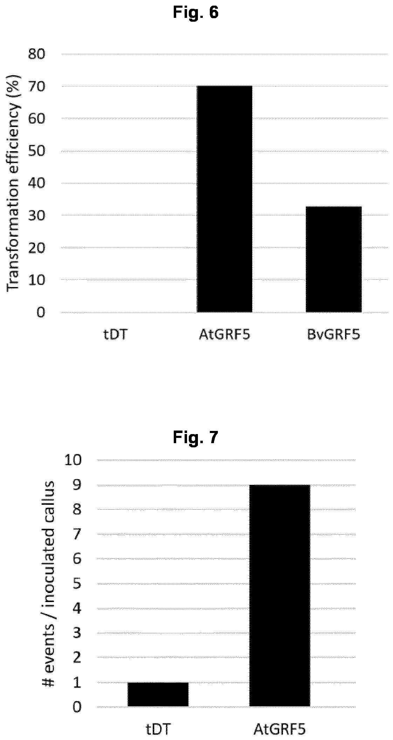

**Fig. 6****:** Transformation frequency in sugar beet obtained with the control construct 70S::tdT, a constructs to overexpress AtGRF5 (cDNA - SEQ ID NO: 1; amino acid sequence - SEQ ID NO: 2), and the sugar beet GRF5 ortholog, BvGRF5 (synthetic DNA - SEQ ID NO: 207; amino acid sequence - SEQ ID NO: 4).
**Fig 7****:** Number of transgenic shoots per inoculated sugar beet callus obtained in transformation experiments by using constructs to overexpress either tdTomato (tDT) (control) and AtGRF5 (cDNA - SEQ ID NO: 1; amino acid sequence - SEQ ID NO: 2).

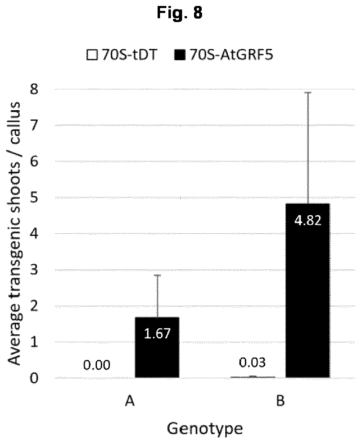

**Fig 8****:** Average number of transgenic shoots per sugar beet callus obtained from transformation experiments either with the control construct 70S-tDT (control) and the construct to overexpress AtGRF5 (70S-GRF5). Two recalcitrant genotypes A and B were used. The recalcitrance level to regenerate shoots of the genotype A is very high, whereas the genotype B displays a milder recalcitrance that A.

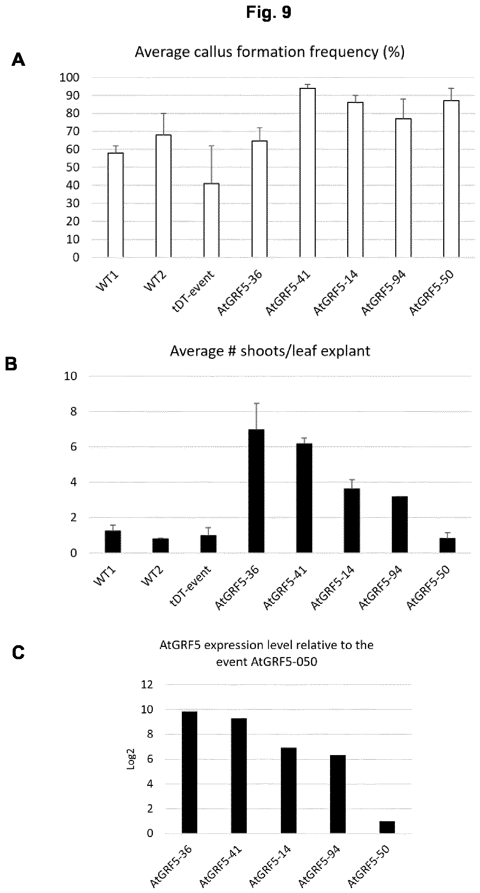

**Fig 9****:** Callus regeneration experiments of transgenic lines overexpressing AtGRF5 in sugar beet. The callus formation frequency **(A)** and the shoot regeneration frequency (**B**) were scored by using the callus induction medium and the shoot regeneration medium described in Kischenko et al., 2005 (see also example 1). A total of 5 independent events overexpressing AtGRF5 were assayed (AtGRF5-36, AtGRF5-41, AtGRF5-14, AtGRF5-94, AtGRF5-50). As control, non-transgenic sugar beet shoots (WT1 and WT2) and transgenic shoots overexpressing the tDT reporter protein (tDT-event) were used. Expression level of AtGRF5 was determined by qRT-PCR on samples isolated from the GRF5 transgenic events used in the assay (**C**).

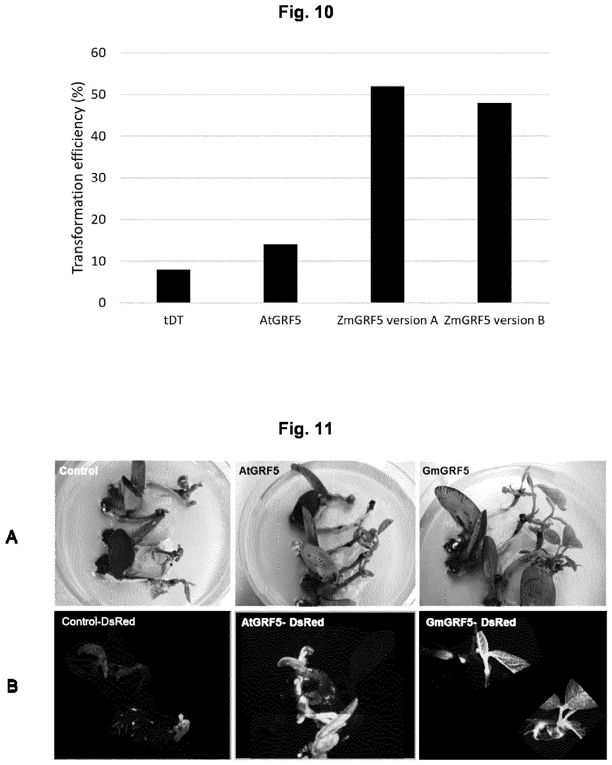

**Fig 10****:** Transformation frequency in Corn, either with the control construct 70S::tDT, or with constructs to overexpress AtGRF5 (cDNA - SEQ ID NO: 1; amino acid sequence - SEQ ID NO: 2), two GRF5 Corn orthologs [ZmGRF5 version A (synthetic DNA - SEQ ID NO: 208; amino acid sequence - SEQ ID NO: 209) and ZmGRF5 version B (synthetic DNA - SEQ ID NO: 210; amino acid sequence - SEQ ID NO: 112). Transformation efficiency was calculated as the number of transgenic events divided by the number of inoculated immature embryos.
**Fig 11****:** Shoot development and DsRed fluorescence of shoots on soybean explants 21 days after transformation. Representative pictures of explants from DsRed control, AtGRF5, and GmGRF5 showing (A) shoot development at the primary-node and (B) DsRed fluorescence of shoots. DsRed pictures are a composite of multiple pictures and positioned in approximate position for orientation purposes.

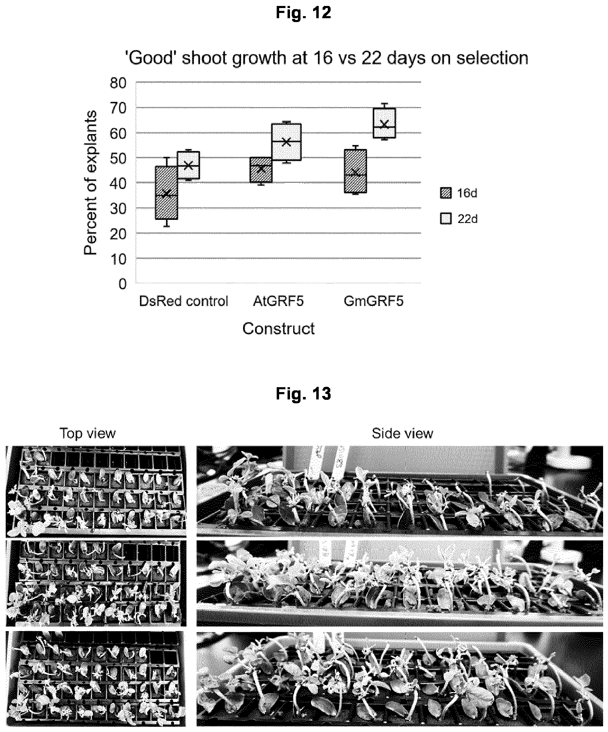

**Fig 12****:** Formation of 'good' shoot growth continued to rapidly increase from 16 to 22 days on selection. The percent of explants were scored at two timepoints to highlight the rapid production of shoots on selection for explants transformed with the three constructs.
**Fig 13****:** Shoot formation on soybean explants transformed with and without GRF5 variants. Pictures taken from top and side view from one repetition is shown for the three treatments (constructs). Shoot growth is more pronounced in the explants transformed with AtGRF5 (middle panel) and GmGRF5 (below panel) vs. the DsRed control (upper panel).

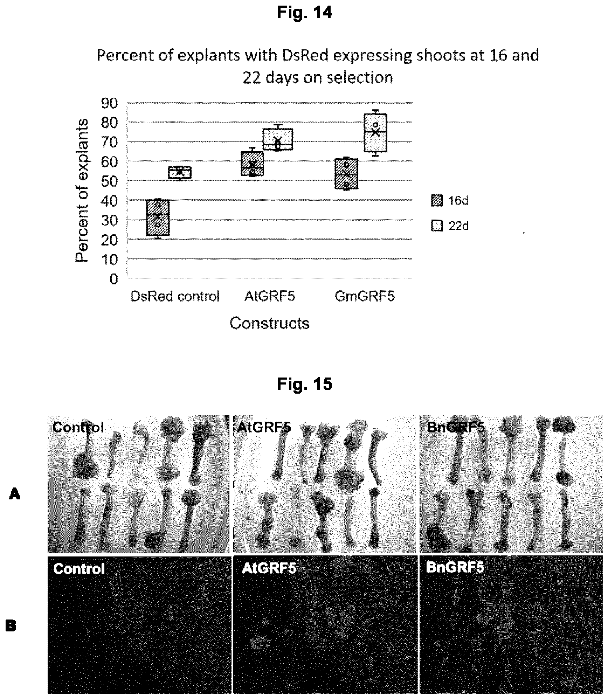

**Fig 14****:** A box-and-whisker plot visualizing the significant increase in regeneration of transgenic shoots in explants transformed with either AtGRF5 or GmGRF5 at 16 and 22 days on selection.
**Fig 15****:** Shoot development and DsRed fluorescence of shoots on canola explants 21 days after transformation. Representative pictures of explants from DsRed control, AtGRF5, and BnGRF5 showing (A) callus development at the hypocotyl segments and (B) DsRed fluorescence on explants.

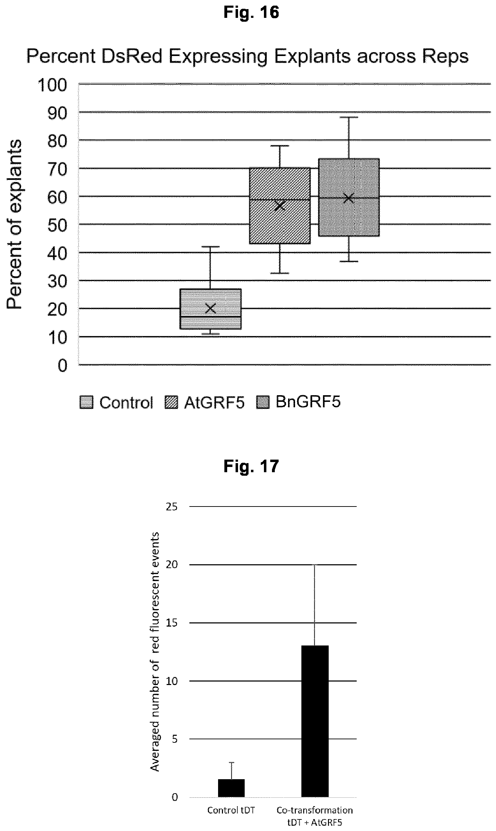

**Fig 16****:** The percent of DsRed expressing explants across five *Brassica napus* experiments transformed with AtGRF5, BnGRF5, and DsRed control are depicted in a box-and-whisker plot. The percent of explants expressing DsRed are significantly greater in AtGRF5 and BnGRF5 compared to control constructs, with nearly a 3-fold increase in the mean.
**Fig 17****:** Co-transformation of sugar beet callus with the control construct 70S-tDT and the 70S-AtGRF5. The inoculation of callus in the co-transformation experiments was done with a mixture 1:1 of the Agrobacterium strains harboring each construct individually.

### Examples

### 1. Beta vulgaris experiments

### Production of the binary plasmid:

The binary vector pZFN-nptll-GRF5 was produced by following standard cloning procedures. Within the T-DNA of this vector, the cDNA encoding GRF5 (At3g13960) was cloned between the double CaMV 35S promoter and the nopaline synthase (NOS) terminator to ensure high ectopic expression levels of the GRF5 protein. The T-DNA also contains the neomycin phosphotransferase II (nptII) gene that confers resistance to a range of aminoglycoside antibiotics such as kanamycin or paromomycin and was used for the selection of transgenic plant cells and tissues. The NOS promoter and the pAG7 terminator flank the nptll gene. The backbone of the binary vector contains the colE1 and the pVS1 origins for plasmid replication in *Escherichia coli* and *Agrobacterium tumefaciens,* respectively; and the aadA gene that confers streptomycin / spectinomycin resistance for bacteria selection. The pZFN-nptli-GRF5 plasmid was transformed into AGL-1 Agrobacterium strain by a standard procedure.

### Transformation of micropropagated shoots:

Shoots of sugar beets were transformed by different methods:
a) Agrobacterium mediated transformation based on Kischenko et al., 2005 Cell Biology International:
   1. Micropropagated shoots of the genotype S706 were used as starting material. Shoots were multiplied in MS salts supplemented with 30 g/l sucrose and 0.25 mg/l benzyladenine (BAP).
   2. To induce friable callus, leaf explants were incubated in MS salts including 15 g/l sucrose and 2 mg/l BAP at around 30°C for several weeks.
   3. Friable calli were harvested.
   4. Agrobacterium AGL-1 harbouring the vector pZFN-nptll-GRF5 was grown in suitable medium supplemented with the appropriate antibiotics.
   5. Calli were inoculated with Agrobacterium suspension. The co-culture of the callus tissue and the Agrobacterium was done in medium containing 440 mg/l CaCl₂x2H₂O, 170 mg/l KH₂PO₄, 1900 mg/l KNO₃, 370 mg/l MgSO₄, 1650 mg/l NH₄NO₃, 2 mg/l BAP, 40 µg/l Acetosyringone, 20 g/l sucrose and 2 g/l glucose for at least 2 days.
   6. Calli were subcultured to MS salts supplemented with 30 g/l sucrose, 1 mg/l GA3, 1 mg/l TDZ and 500 mg/l Timentin and incubated in the dark, for c. 1 week.
   7. For the selection of transgenic cells, calli were transferred to the medium of step 6 supplemented with 100 mg/l paromomycin and incubated in the light for several weeks.
   8. Transgenic calli were selected and subcultured for several times in the same medium and conditions.
   9. Regenerating shoots were isolated and propagated in MS salts including 30 g/l sucrose, 0.25 mg/l BAP and 100 mg/l kanamycin.
   10. Green shoots were transferred to MS salts supplemented with 30 g/l sucrose, 0.1 mg/l BAP, 250 mg/l Timentin and 200 mg/l paromomycin in order to finish the selection phase and were multiplied regularly for several times in this media.
   11. Leaf explants were isolated from the green growing shoots for DNA extraction and PCR analysis, in order to confirm the putative transgenic lines.
   12. Selected shoots were rooted in MS salts supplemented with 0.5 mg/l IBA, 100 mg/l cefotaxime and 10 mg/l PPT and transferred to the green house for seed production.
b) Particle bombardment of sugar beet callus
   1. Friable calli were produced as previously described in the method of 2.1.
   2. An osmotic treatment was carried out for several hours.
   3. Preparation and DNA coating of the gold particles was done by standard procedures, as describe in the PDS-1000/He instruction manual. The plasmids pZFN-nptll-GRF5 and pUbi4-tDT (containing a red fluorescent reporter) were coprecipitated with gold particles. As control, gold particles were coated with pUbi4tDT and pABM70STurboYFP (containing a yellow fluorescent reporter).
   4. Calli were bombarded with a PDS-1000/He unit (Bio-Rad), using 30 ng gold particles coated with 500 ng DNA per shot.
   5. To evaluate the effect of the transient expression of GRF5 in the shoot regeneration frequency, bombarded calli were incubated in MS salts supplemented with 30 g/l sucrose, 1 mg/l GA3 and 1 mg/l Thidiazuron in light conditions for c. 2 weeks. Shoot number was scored by using a standard binocular.

### Results:

The *Agrobacterium tumefaciens*-mediated transformation of calli derived from micropropagated shoots of sugar beet with the construct pZFN-nptll-GRF5 increases the number of regenerated shoots at the end of the selection step significantly (Table 2). As control, the construct pZFN-tdT-nptll (Control) containing a red fluorescent reporter has been used. The five independent experiments show an increase of the on average transformation frequency from 5.0% to 33.9%. Further the number of transgenic events confirmed by PCR has been increased on average from 4.8 events to 25.4 events per transformation experiment.

**Table 2. Transformation frequency of 5 independent experiments performed by the transformation method of a), either with a control construct or with the pZFN-nptll-GRF5. The total number of shoots at the end of the selection phase and total number of transgenic events confirmed by PCR are also shown. On average number of developing shoots, transgenic events and transformation frequency are indicated in bold.**

| Experiment Name | # shoots at the end of selection | # confirmed transgenic lines | Transformation frequency (%) |
|---|---|---|---|
| Control_Rep01 | 9 | 3 | 5.5 |
| Control_Rep02 | 8 | 5 | 8.5 |
| Control_Rep03 | 11 | 5 | 3.3 |
| Control_Rep04 | 9 | 4 | 3.1 |
| Control_Rep05 | 13 | 7 | 4.7 |
| **Control average** | **10** | **4.8** | **5.0** |
| GRF5_Rep01 | 108 | 23 | 11.5 |
| GRF5_Rep02 | 61 | 28 | 35.0 |
| GRF5_Rep03 | 27 | 13 | 21.7 |
| GRF5_Rep04 | 167 | 35 | 38.9 |
| GRF5_Rep05 | 86 | 28 | 62.2 |
| **GRF5 average** | **89.8** | **25.4** | **33.9** |

In the callus transformation experiments the regeneration of shoots has been determined also for each individual selection step. The Quantification of regenerating shoots in each selection step shows that the overexpression of the GRF5 in calli of sugar beet accelerates the shoot organogenesis (Table 3, figures 1 and 2). In another callus transformation experiment the regeneration of shoots has been determined for two recalcitrant sugar beet genotypes (Figure 8): The transformation with construct pZFN-tdT-nptll (control) results in no transgenic shoot for genotype A and in only on average 0.3 shoots per callus for genotype B. Using AtGRF5 overexpression under the 70S promoter the average number of transgenic shoots is increased from 0 to 1.67 transgenic shoots for genotype A and from 0.3 to 4.82 shoots per callus for genotype B. This demonstrate impressively the potential of AtGRF5 and opens up the possibility to work genotype-independently with standard transformation methods.

Further experiments demonstrated that the number of transgenic events per inoculated callus could be increased by factor 9 by use of a construct for overexpression of AtGRF5 in *Beta vulgaris* calli (Figure 9). As control, the construct pZFN-tdT-nptll (Control) containing a red fluorescent reporter has been used. The GRF gene as well as the reporter gene were under the control of the constitutive 70S promoter (double enhanced constitutive 35S promoter from Cauliflower mosaic virus).

The expression level of GRF5 has been determined in eleven, randomly chosen independent transgenic events of sugar beet. The expression analysis was performed with primers binding to the 3'-UTR of the NOS terminator. In all analyzed transgenic events a high level of GRF5 expression have been detected (see figure 4).

**Table 3. Quantification of regenerating shoots in each selection step of 5 independent callus transformation experiments done either with pZFN-tdT-nptll (Ctrl) or pZFN-nptll-GRF5 (RB). Total number of developing shoots in each step are indicated in bold.**

| | | SELECTION STEPS | | | |
|---|---|---|---|---|---|
| Experiment ID | Overexpressed gene | S1 | S2 | S3 | S4 |
| Ctrl-1 | tdTomato | 0 | 0 | 3 | 6 |
| Ctrl-2 | tdTomato | 0 | 1 | 3 | 4 |
| Ctrl-3 | tdTomato | 0 | 0 | 0 | 11 |
| Ctrl-4 | tdTomato | 0 | 0 | 4 | 5 |
| Ctrl-5 | tdTomato | 0 | 0 | 7 | 6 |
| **Ctrl-Total** | **tdTomato** | **0** | **1** | **17** | **32** |
| RB-1 | GRF5 | 0 | 40 | 41 | 27 |
| RB-2 | GRF5 | 0 | 11 | 20 | 30 |
| RB-3 | GRF5 | 0 | 9 | 11 | 7 |
| RB-4 | GRF5 | 0 | 65 | 77 | 25 |
| RB-5 | GRF5 | 2 | 27 | 23 | 34 |
| **RB-Total** | **GRF5** | **2** | **152** | **172** | **123** |

In a further experiment of callus regeneration five different transgenic events overexpressing AtGRF5 in sugar beet have been analyzed. As control, on the one hand two non-transgenic sugar beet lines (WT1 and WT2) and on the other hand a transgenic line overexpressing the tdT reporter protein were used. Figure 9 A shows that the average callus formation frequency in the control lines and the transgenic AtGRF5 events were almost comparable, perhaps slightly enhanced in the transgenic events. In Figure 9B the average number of transgenic shoots is presented. Four of the five AtGRF5 events show a significant increase in shoot formation compared the two controls. From Figure 9 C is becomes apparently that the positive effect on shoot formation is related to the expression level of AtGRF5 in the transgenic events.

In co-transformation experiments with the control construct 70S-tDT and the 70S-AtGRF5 has been stably integrated in the genome of sugar beet callus cells. The inoculation of callus in the co-transformation experiments was done with a mixture 1:1 of the Agrobacterium strains harboring each construct individually. The average co-transformation frequency was 31.5%. As shown in Figure 17, the number of red fluorescent (tDT positive) events is around 8 times higher in the co-transformation than in the single transformation experiments.

The transformation via particle bombardment according to method of 2.2 showed that even the transient (over)expression of GRF5 results in a significant increase of transformation frequency. Bombarded calli show an improved regeneration capability. Figure 3 shows that calli biolistically transformed with construct pZFN-nptll-GRF5 exhibit an increased number of regenerating shoots compared to calli with the control construct.

In additional callus transformation experiments the transformation frequency in sugar beet has been determined also using the construct pZFN-nptll-AtGRF5 compared with the same construct but expressing the GRF homolog from Beta vulgaris (BvGRF5). As control, the construct pZFN-tdT-nptll (Control) containing a red fluorescent reporter has been used. The GRF genes as well as the reporter gene were under the control of a constitutive 70S promoter. The experiments shown in Figure 6 were stopped at an early selection phase. That explains why transformation efficiency of the tDT construct is 0%. For the construct AtGRF5 on average transformation frequency of 70% and for construct BvGRF5 on average transformation frequency of 32.5% could be achieved.

### 2. Oryza sativa experiments

### Constructs used in the binary plasmid:

The binary vectors were produced by standard cloning procedures. Within the T-DNA of this vector, the cDNA encoding GRF5 (At3g13960) was cloned between a suitable promoter and terminator ensuring sufficient ectopic expression levels of the GRF5 protein in rice (=single constructs). The T-DNA also contains the GFP gene that was used for the selection of transgenic plant cells and tissues. Additionally, binary vectors were produced which carrying beside the cDNA encoding GRF5 (At3g13960) including the suitable promoter and terminator ensuring sufficient ectopic expression levels of the GRF5 protein in rice and a further gene of interest under the control of a promoter and terminator (=double (stack) constructs).

### Seed sterilization and sowing:

Wild type green seeds have been incubated in 70% ethanol and shaked for approximately 1 minute. After removal of ethanol the seeds have been washed once with sterile mQ water. Then 30 ml of 6% sodium hypochlorite solution has been added and the seeds have been shaked for 40-60 minutes. After removal of the sodium hypochlorite solution seeds have been washed 3 to 5 times with sterile mQ.

After finishing sterilization (0-3 hours) the seeds have been dried on sterile filter paper and placed onto the surface of the induction medium R001. The incubation took place under continuous light (3000 lux) at 32°C for 6 days.

### Transformation & Co-cultivation:

For explant preparation swollen embryo's (scutellum derived calli) from the wild type seeds suitable for transformation has been selected and transferred to liquid infection medium (R002) containing *Agrobacterium tumefaciens* transformed with the plasmid being incorporated into the plant cells for 1.5 minutes and then to the cocultivation plates (R003). The plates have been incubated for 3 days at 25°C in darkness. Selection of resistant tissue

### Selection:

After 3 days on cocultivation the calli have been removed from the seeds, washed several times with sterile mQ water and once with sterile mQ water containing 250 mg/l cefotaxime and transferred to R004 selection medium. Incubation has been performed under continuous light (3000 lux) at 32°C for 2 weeks.

### Microcalli isolation & Regeneration

By means of sterile forceps the microcalli have been transferred to R005 and incubated under continuous light (3000 lux) at 32°C for 1 week. Thereafter GFP used as selection marker has been checked in the dark room. Healthy calli positive for GFP have been transfer to R006 and further incubated under continuous light (3000 lux) at 32°C for 1 week. For continuous regeneration, the calli have been transferred to R007 for 3 weeks under continuous light (3000 lux) at 32°C. With sterile forceps, healthy plantlets have been pulled out and transferred to R008 for 2 weeks under continuous light (3000 lux) at 32°C before the plantlets have been brought to the greenhouse.

**Table 4. Composition of media R001 to R008.**

| **R001 (solid) Induction medium** | | | | |
|---|---|---|---|---|
| **ingredients** | **supplier/stock code** | **final concentration** | **1.00** | **l** |
| N6 salts | Sigma-C1416 | | 4.00 | g |
| N6 vitamins | Duchefa - C0401 | 1x | 1.00 | ml |
| L-Proline | Sigma -P5607 | 2878 mg/l | 2.88 | g |
| CasaminoAcids | BD - 223050 | 300 mg/l | 0.30 | g |
| Sucrose | Duchefa - S0809 | 30 g/l | 30.00 | g |
| pH | | | 5.80 | |
| Gelrite | Duchefa - G1101.5 | 4 g/l | 4.00 | g |
| 2,4-D (1mg/ml) | Sigma - D7299 | 2 mg/l | 2000.00 | µl |
| | | | | |

| **R002 (liquid) Infection medium (filter sterilized)** | | | | |
|---|---|---|---|---|
| **ingredients** | **supplier/stock code** | **final concentration** | **1.00** | **l** |
| N6 salts | Sigma - C1416 | 1x | 4.00 | g |
| N6 vitamins | Duchefa - C0401 | 1x | 1.00 | ml |
| CasaminoAcids | BD - 223050 | 300 mg/l | 0.30 | g |
| Sucrose | Duchefa - S0809 | 68.5 g/l | 68.50 | g |
| D+-Glucose-Monohydrat | VWR - MERC1.08342.1000 | 36 g/l | 36.00 | g |
| pH | | | 5.20 | |
| acetosyringone (2M) | Sigma Aldrich 2478-38-8 | 100 µM | 66.00 | µl |
| *preparation of acetosyringone: 2M=392 mg in 1 ml DMSO dus: 0.04 g in 100 µl DMSO* | | | | |
| | | | | |

| **R003 (solid) Co cultivation medium** | | | | |
|---|---|---|---|---|
| **ingredients** | **supplier/stock code** | **final concentration** | **1.00** | **l** |
| N6 salts | Sigma - C1416 | | 4.00 | g |
| N6 vitamins | Duchefa - C0401 | 1x | 1.00 | ml |
| CasaminoAcids | BD - 223050 | 300 mg/l | 0.30 | g |
| Sucrose | Duchefa - S0809 | 30 g/l | 30.00 | g |
| D+-Glucose-Monohydrat | VWR - MERC1.08342.1000 | 10 g/l | 10.00 | g |
| pH | | | 5.20 | |
| Gelrite | Duchefa - G1101.5 | 4 g/l | 4.00 | g |
| acetosyringone (2M) | Sigma Aldrich 2478-38-8 | 100 µM | 66.00 | µl |
| 2,4-D (1mg/ml) | Sigma - D7299 | 2 mg/l | 2000.00 | µl |
| *preparation of acetosyringone: 2M=392 mg in 1 ml DMSO dus: 0.04 g in 100 µl DMSO* | | | | |
| | | | | |

| **R004 selection medium (LBA nptll)** | | | | |
|---|---|---|---|---|
| **ingredients** | **supplier/stock code** | **final concentration** | **1.00** | **l** |
| N6 salts | Sigma - C1416 | | 4.00 | g |
| N6 vitamins | Duchefa - C0401 | 1x | 1.00 | ml |
| L-Proline | Duchefa - P0717 | 2878 mg/l | 2.88 | g |
| CasaminoAcids | BD - 223050 | 300 mg/l | 0.30 | g |
| Sucrose | Duchefa - S0809 | 30 g/l | 30.00 | g |
| pH | | | 5.80 | |
| agarose type 1 | Sigma - A6013 | 7 g/l | 7.00 | g |
| 2,4-D (1mg/ml) | Sigma - D7299 | 2 mg/l | 2000.00 | µl |
| Cefotaxime (200 mg/ml) | Duchefa - C0111 | 100 mg/l | 500.00 | µl |
| Vancomycin (100 mg/ml) | Duchefa - V0155 | 100 mg/l | 1000.00 | µl |
| G418 disulfate (100 mg/ml) | Sigma - G1279 | 35 mg/l | 350.00 | µl |
| | | | | |

| **R005 Pre-regeneration medium** | | | | |
|---|---|---|---|---|
| **ingredients** | **supplier/stock code** | **final concentration** | **1.00** | **l** |
| N6 salts | Sigma - C1416 | | 4.00 | g |
| N6 vitamins | Duchefa - C0401 | 1x | 1.00 | ml |
| L-Proline | Duchefa - P0717 | 500 mg/l | 0.50 | g |
| CasaminoAcids | BD - 223050 | 300 mg/l | 0.30 | g |
| Sucrose | Duchefa - S0809 | 30 g/l | 30.00 | g |
| pH | | | 5.80 | |
| agarose type 1 | Sigma - A6013 | 7 g/l | 7.00 | g |
| Kinetin (1mg/ml) | Sigma - K0753 | 2 mg/l | 2000.00 | µl |
| NAA (1mg/ml) | Duchefa - N0903 | 1 mg/l | 1000.00 | µl |
| ABA | Sigma - A 1049 | 5 mg/ml | 1000.00 | µl |
| Cefotaxime (200 mg/ml) | Duchefa - C0111 | 100 mg/l | 500.00 | µl |
| Vancomycin (100 mg/ml) | Duchefa - V0155 | 100 mg/l | 1000.00 | µl |
| G418 disulfate (100 mg/ml) | Sigma - G1279 | 35 mg/l | 350.00 | µl |
| | | | | |

| **R006 Regeneration medium with 10 g/l agarose** | | | | |
|---|---|---|---|---|
| **ingredients** | **supplier/stock code** | **final concentration** | **1.00** | **l** |
| MS salts | Duchefa - M0221 | 1x | 4.30 | g |
| MS vitamins | Duchefa - 1000x /M0409 | 1x | 1.00 | ml |
| CasaminoAcids | BD - 223050 | 2000 mg/l | 2.00 | g |
| Sucrose | Duchefa - S0809 | 30 g/l | 30.00 | g |
| Sorbitol | Duchefa S0807 | 30 g/l | 30.00 | g |
| pH | | | 5.80 | |
| agarose type 1 | Sigma - A6013 | 10 g/l | 10.00 | g |
| Kinetin (1mg/ml) | Sigma - K0753 | 2 mg/l | 2000.00 | µl |
| NAA (1mg/ml) | Duchefa - N0903 | 0.02 mg/l | 20.00 | µl |
| Cefotaxime (200 mg/ml) | Duchefa - C0111 | 100 mg/l | 500.00 | µl |
| Vancomycin (100 mg/ml) | Duchefa - V0155 | 100 mg/l | 1000.00 | µl |
| G418 disulfate (100 mg/ml) | Sigma - G1279 | 20 mg/l | 200.00 | µl |
| | | | | |

| **R007 Regeneration medium** | | | | |
|---|---|---|---|---|
| **ingredients** | **supplier/stock code** | **final concentration** | 1.00 | **l** |
| MS salts | Duchefa - M0221 | 1x | 4.30 | g |
| MS vitamins | Duchefa - 1000x IM0409 | 1x | 1.00 | ml |
| Sucrose | Duchefa - S0809 | 30 g/l | 30.00 | g |
| pH | | | 5.80 | |
| agarose type 1 | Sigma - A6013 | 7 g/l | 7.00 | g |
| Cefotaxime (200 mg/ml) | Duchefa - C0111 | 100 mg/l | 500.00 | µl |
| Vancomycin (100 mg/ml) | Duchefa - V0155 | 100 mg/l | 1000.00 | µl |
| G418 disulfate (100 mg/ml) | Sigma - G1279 | 20 mg/l | 200.00 | µl |
| | | | | |

| **R008 Development medium** | | | | |
|---|---|---|---|---|
| **ingredients** | **supplier/stock code** | **final concentration** | **1.00** | **l** |
| MS salts | Duchefa - M0221 | 2.151045 g/l | 2.15 | g |
| B5 vitamins (1000x stock) | Duchefa - G0415 | 0.5x | 0.50 | ml |
| Sucrose | Duchefa - S0809 | 10 g/l | 10.00 | g |
| NAA | Duchefa - N0903 | 0.05 mg/l | 50.00 | µl |
| MgCl2.6H2O | VWR - MERC1 | 0.75 g/l | 0.75 | g |
| pH | | | 5.80 | |
| Gelrite | Duchefa - G1101.5 | 2.5 g/l | 2.50 | g |

### Results:

The *Agrobacterium tumefaciens*-mediated transformation of calli derived from immature embryos of rice with the different constructs containing GRF5 leads to a significant increase of the on average transformation frequency. As control or as comparison the transformation efficiency observed for 28 randomly selected other constructs without GRF5 has been used that shows a on average transformation efficiency of 63%. For 'single' constructs the transformation efficiency could be increased on average from 63% to 78%, for 'double' constructs even from 63% to 84% (Table 5).

**Table 5. Transformation frequency of independent experiments, either with a single construct with GRF5 or with GRF5 in a stack with another gene XY in Rice. In total, 12 different genes in the stack with GRF5 from Arabidopsis thaliana ("GRF5", nucleotide sequence as set forth in SEQ ID NO: 1, amino acid sequence of SEQ ID NO: 2) have been tested, whereby 3 experiments have been repeated two times (indicated by Rep01 and Rep02). Also, GRF5 from Sorghum bicolor ("GRF5-Sorghum", nucleotide sequence as set forth in SEQ ID NO: 17, amino acid sequence of SEQ ID NO: 18) was tested alone and in a stack. On average transformation frequency are indicated in bold.**

| Single/Stack | Gene #1 | Gene #2 | Transformation frequency [%] |
|---|---|---|---|
| Single | GRF5 | | 72 |
| Single | GRF5 | | 62 |
| Single | GRF5 | | 73 |
| Single | GRF5 | | 88 |
| Single | GRF5 | | 93 |
| | | **Average:** | **78** |
| | | | |
| Double | GRF5 | Gene XY-01 | 77 |
| Double | GRF5 | Gene XY-02 | 86 |
| Double | GRF5 | Gene XY-03_Rep01 | 91 |
| Double | GRF5 | Gene XY-03_Rep02 | 92 |
| Double | GRF5 | Gene XY-04 | 92 |
| Double | GRF5 | Gene XY-05_Rep01 | 75 |
| Double | GRF5 | Gene XY-05_Rep02 | 100 |
| Double | GRF5 | Gene XY-06 | 79 |
| Double | GRF5 | Gene XY-07 | 89 |
| Double | GRF5 | Gene XY-08 | 79 |
| Double | GRF5 | Gene XY-09 | 81 |
| Double | GRF5 | Gene XY-10_Rep01 | 80 |
| Double | GRF5 | Gene XY-10_Rep02 | 82 |
| Double | GRF5 | Gene XY-11 | 76 |
| Double | GRF5 | Gene XY-12 | 77 |
| | | **Average:** | **84** |
| | | | |
| Single | GRF5-Sorghum | | 67 |
| | | **Average:** | **67** |
| | | | |
| Double | GRF5-Sorghum | Gene XY-13 | 78 |
| | | **Average:** | **78** |
| | | | |
| Control: | | **Average:** | **63** |

### 3. Zea mays experiments

### Constructs used in the binary plasmid:

The binary vectors were produced by standard cloning procedures. Within the T-DNA of these vectors, a) the cDNA encoding AtGRF5 (SEQ ID NO: 1), b) the synthetic DNA encoding ZmGRF5 (version A) (SEQ ID NO: 208), and c) the synthetic DNA encoding ZmGRF5 (version B) SEQ ID NO: 210) were cloned between a suitable promoter (e.g. BdEF1 promoter) and terminator ensuring sufficient ectopic expression levels of the GRF5 proteins in corn. As control a construct containing the tDT reporter gene under control of the 70S promoter with the ZmUbi Intron has been used.

### Transformation:

The Agrobacterium *tumefaciens*-mediated transformation has been conducted by standard method of transforming monocotyledon by using scutellum of immature embryo (e.g., WO 95/06722).

### Results:

As shown in Figure 10 the *Agrobacterium* tumefaciens-mediated transformation of immature embryos with construct containing the GRF derived from *Arabidopsis thaliana* leads to a significant increase of the on average transformation frequency, from 8% to 14%. In contrast thereto, the use of the both ZmGRF5 versions derived from different *Zea mays* genotypes boosted the transformation efficiency much stronger. For version A of ZmGRF5 the efficiency is increased from 8% to 52%, and for version B of ZmGRF5 from 8% to 48%.

### 4. Glycine max (Soybean) experiments

### Soybean Transformation:

*Glycine max* transformation was performed using *Agrobacterium rhizogenes* for T-DNA delivery into the epicotyl's axillary meristem cells located at the primary-node of soybean seedlings of cultivar Jake (according to Olhoft P.M., Bernal L.M., Grist L.B., Hill D.S., Mankin S.L., Shen Y., Kalogerakis M., Wiley H., Toren E., Song H.-S., Hillebrand H., and Jones T. 2007, A novel Agrobacterium rhizogenes-mediated transformation method soybean [Glycine max (L.) Merrill] using primary-node explants from seedlings, In Vitro Cell. Dev. Biol.-Plant 43:536-549; US 2014237688, WO 2006024509, WO 2005121345).

### Production of binary plasmids:

Three binary plasmids were produced by following standard cloning procedures. The first binary plasmid was the control plasmid used for the experiments (referred to the DsRed control) and is the base vector used for the other vectors. It contains within the T-DNA a DsRed gene that was used for phenotypic scoring of transgenic plant cells and tissues and the AtAHAS gene that was used for preferential selection of transgenic cells. Both genes were cloned with suitable promoter and terminators that ensure sufficient ectopic expression to serve abovementioned purposes. The second binary plasmid contains the base vector with the cDNA encoding AtGRF5 (SEQ ID NO. 2) cloned between a suitable promoter and terminator ensuring sufficient ectopic expression levels of the GRF5 protein in soybean. The third binary plasmid contains the base vector with the cDNA encoding GmGRF5 (SEQ ID NO. 106) cloned between a suitable promoter and terminator ensuring sufficient ectopic expression levels of the GRF5 protein in soybean.

### Seed Sterilization and Germination:

Soybean seeds of 'Jake' cultivar were sterilized in a chamber with a chlorine gas produced by adding 3.5 ml 12N HCl into 100 ml bleach. After sterilization, approximately 65 seeds were plated on solid germination medium (1X B5 salts and vitamins, 2% sucrose, 0.8% Noble agar (A5431 Sigma-Aldrich^{®}); pH 5.8) in PlantCons^{™}. The seedlings were germinated in the light (150 µm⁻²s⁻²) at 26°C for 7 days and used as explant material for transformation.

### Agrobacterium Preparation:

*Agrobacterium rhizogenes* (WO 2006024509) was transformed with one of the following vectors containing: (1) pSUPER:DsRed and pPcUBI:AHAS selectable marker as a control, or the control vector plus either (2) pPcUbi-AtGRF5 or (3) pPcUBI-GmGRF5. *A. rhizogenes* was grown and resuspended in 50 ml liquid inoculation medium (1/10^{th} B5 salts (G768 Phytotech), 3% sucrose, 20 mM MES, 1X Gamborg's vitamins, 200 µM acetosyringone, 1.44 µM gibberellic acid, 5.0 µM Kinetin; pH 5.4.) in a Falcon tube to an OD₆₀₀ of 1.5. The *Agrobacterium* suspension was then placed in a deep petri dish for receiving prepared explants.

### Explant Preparation and Transformation:

Seedling explants were prepared from the 8-day-old seedlings by removing the roots and majority of the hypocotyl, one cotyledon, the axillary tissue growth at cotyledonary node, and the epicotyl above primary-node including all preformed leaves. After preparing the explants, approximately 45-50 explants were incubated with the *Agrobacterium* suspension in the petri dish for 30 minutes. Explants were then placed in petri dishes on a wet filter paper containing co-cultivation medium (1/10^{th} B5 salts (G768 Phytotech), 3% sucrose, 20 mM MES, 0.5% Noble agar (A5431 Sigma-Aldrich^{®}), 1X Gamborg's vitamins, 200 µM acetosyringone, 1.44 µM gibberellic acid, 5.0 µM kinetin, 4.1 mM L-cysteine, 0.5 mM dithiothrietol, 0.5 mM sodium thiosulfate; pH 5.4), and sealed in a container for 5 days at room temperature.

### Shoot Development and Selection:

After 5 days, explants were transferred to selection medium (1X B5 salts and vitamins (G398 Phytotech), 3% sucrose, 3 mM MES, 1 µM 6-benzyl-aminopurine, 5 µM Kinetin, 250 mg/l Timentin STK, 3 µM imazapyr, 0.8% Noble agar (A5431 Sigma-Aldrich^{®}); pH 5.6) five per plate and cultivated at 26°C. The explants had significant growth at the primary-node axillary meristem after 16 days on selection and were first scored for shoot development (regeneration). After 22 days on selection (the end of selection), the explants were removed from the solid media and placed on Oasis^{®} growing media prior to scoring for (1) quality of shoot formation and (2) DsRed fluorescence on shoots developing at the primary-node.

### Experimental Design and results:

One experiment was conducted across four researchers and three constructs (Table 6). Soybean primary-node axillary meristems were transformed with DsRed control, AtGRF5, and GmGRF5. In total, 524 seedling explants were transformed and scored after 16 days on selection (21 days after transformation) and 22 days on selection (27 days after transformation). After 16 days on selection, shoots were rapidly growing at the primary-node and were a combination of small, compact shoot pads and larger, elongating shoots (Figure 11). Regeneration [(number of explants with shoots)/total explants*100] at the target tissue, the primary-node, was between 80-100% for all constructs, and was fixed at 16 days on selection. Between 16 and 22 days, the shoots on the explants continued to grow rapidly. For both timepoints, the explants were subjectively scored for the presence of healthy, elongated shoot growth with a morphology ('good') that is predictive of successful formation of a rooted, transgenic plant (Figure 12). There was an increase in 'good' shoot formation on explants transformed with all three constructs from the two timepoints, especially for explants transformed with AtGRF5 and GmGRF5. Across the four replicates, the explants transformed with either form of GRF5 tended to have more advanced shoot formation (larger shoots, more elongated) when compared to the DsRed control (Figure 13).

To get an early measurement of transformation efficiencies, the explants were scored at 16 and 22 days on selection for the presence or absence of DsRed fluorescing shoots at the primary-node, which is due to transgenic cells expressing the DsRed protein. The DsRed expression was markedly more intense in the constructs containing AtGRF5 or GmGRF5 than the DsRed control at both timepoints; however more so at 16 days on selection (Figure 11). The explants that were characterized as having 'good' shoot morphology generally had shoots that had DsRed expression (Figure 11). The percent of explants with DsRed expressing shoots was significantly greater (at α=0.05) in explants that were transformed with either AtGRF5 or GmGRF5 than compared to explants transformed with the DsRed control at both timepoints (Table 6; Figure 14). Of the explants transformed with the DsRed control construct, 54.5% showed DsRed expressing shoots compared to 70.2% and 74.6% for AtGRF5 and GmGRF5, respectively, after 22 days on selection (Table 6). The data indicate that at 27 days after transformation, soybean explants transformed with either AtGRF5 or GmGRF5 have significantly more transgenic shoots regenerating at the primary-node than explants not transformed with either form of GRF5.

**Table 6. Regeneration of transformed shoots was significantly increased in explants transformed with either AtGRF5 or GmGRF5 at 16 days and 22 days on selection. The mean and the range in regeneration and DsRed fluorescing shoots are shown. Constructs that were significantly different (at α=0.05) are followed with different letters; ¹= [(number of explants with shoots at primary-node/total number {n} of explants inoculated) × 100]; ²= [(number of explants with DsRed shoots at primary-node/total number {n} of explants inoculated) × 100]**

| Construct | Regeneration (%)¹ | | | Explants (%) with DsRed shoots² | | | |
|---|---|---|---|---|---|---|---|
| | *n* | 16d on selection | | 16d on selection | | 22d on selection | |
| | | Mean | Range (%) | Mean | Range (%) | Mean | Range (%) |
| DsRed control | 162 | 89.8 a | 81.8-95.2 | 31.4 a | 20.5-40.5 | 54.5 a | 50.0-57.1 |
| AtGRF5 | 180 | 88.4 a | 82.6-91.3 | 58.0 b | 52.2-66.7 | 70.2 b | 65.2-78.6 |
| GmGRF5 | 182 | 94.6 a | 85.4-100 | 53.3 b | 45.2-61.9 | 74.6 b | 62.5-86.0 |

### 5. Brassica napus (Canola) experiments

### Canola Transformation:

*Brassica napus* transformation was performed using *Agrobacterium rhizogenes* for T-DNA delivery into hypocotyl segments of *B. napus* seedlings of genotype BNS3.

### Seed Sterilization and Germination:

Seeds were surface sterilized by placing 200-300 seeds for 2 minutes in 70% ethanol in a 50-mL Falcon tube. After gently shaking for 2 minutes, the ethanol was removed and 40 to 50 ml 30% Clorox bleach with 1 drop Tween was added. The seeds were incubated for 10 minutes with occasional mixing. The liquid was removed, then the seeds rinsed with sterile water three times before placing seeds on germination medium (1/2x MS salts/ vitamins, 10 g I⁻¹ sucrose, Phyto Agar 7g I⁻¹; pH 5.8) in PlantCon boxes. The boxes were placed in a chamber for 4 to 5 days in the dark at 23°C.

### Agrobacterium Preparation:

*Agrobacterium rhizogenes* was transformed with one of the following vectors: (1) pSUPER:DsRed and PcUBI:AHAS selectable marker as a control, or the control vector plus either (2) PcUbi-AtGRF5 or (3) PcUBI-BnGRF5 (SEQ ID NO. 108). *Agrobacterium rhizogenes* was grown to OD₆₀₀ of 1.0 and subsequently diluted to 0.1 with liquid infection medium (1x MS salts/vitamins, 30 g I⁻¹ sucrose; pH 5.8). The *Agrobacterium* suspension was then placed in a dish for receiving prepared hypocotyl explants.

### Explant Preparation and Transformation:

Hypocotyl explants were prepared from the four to five-day etiolated seedlings by removing from germination box and placing on sterile filter paper wetted with infection medium without *Agrobacterium* to keep seedlings turgid. Hypocotyl segments 7 to 10 mm in length were prepared by after removing the root, cotyledon and epicotyl. After cutting, the explant was dipped in the *Agrobacterium* suspension, blotted on dry, sterile filter paper, and finally placed on filter paper on top of co-culture medium (1x MS salts/vitamins, 30 g I⁻¹ sucrose, 0.6 g I⁻¹ MES, 18 g I⁻¹ mannitol, 7 g I⁻¹ Phyto Agar, 1 mg I⁻¹ 2,4-D, 100 mg I⁻¹ acetosyringone, 200 mg I⁻¹ L-cysteine; pH 5.6) in plates. Once ~50 explants were plated, the plates were sealed with micropore tape and cultivated in the light at 23°C at a 16 h light/8 h dark photoperiod for 3 days.

### Callus Development and Shoot Initiation:

After 3 days, all explants were transferred to recovery medium (1x MS salts/vitamins, 30 g I⁻¹ sucrose, 0.6 g I⁻¹ MES, 18 g I⁻¹ mannitol, 7 g I⁻¹ Phyto Agar, 1 mg I⁻¹ 2,4-D, 300 mg I⁻¹ Timentin; pH 5.6), sealed with micropore tape, and cultivated at 23°C for 7 days.

Explants were transferred to selection medium #1 (1x MS salts/vitamins, 30 g I⁻¹ sucrose, 0.5 g I⁻¹ MES, 7 g I⁻¹ Phyto Agar, 3 mg I⁻¹ BAP, 0.1 mg I⁻¹ NAA, 0.1 mg I⁻¹ GA₃, 2.5 mg I⁻¹ AgNO₃, 100 nM imazethapyr, 300 mg I⁻¹ Timentin; pH 5.8) after one week, and cultivated for 14 days at 23°C under 16 h light/8 h dark photoperiod. After two weeks of selection, the explants were transferred to Selection Medium 2 (1x MS salts/vitamins, 30 g I⁻¹ sucrose, 0.5 g I⁻¹ MES, 7 g I⁻¹ Phyto Agar, 0.5 mg I⁻¹ BAP, 0.1 mg I⁻¹ GA₃, 2.5 mg I⁻¹ AgNO₃, 100 nM imazethapyr, 300 mg I⁻¹ Timentin; pH 5.8).

### Experimental design and results:

Five experiments over time were conducted across 3 researchers with a minimum of one replicate per researcher (Table Canola-1). In experiment 1, *Brassica napus* hypocotyls were transformed with DsRed control and BnGRF5 constructs for experiment 1 only; in the other experiments, explants were transformed with DsRed control, AtGRF5, and BnGRF5 constructs. In total, 3,156 explants were inoculated and scored for DsRed fluorescence after 16 to 22 days. At this point in time, explants were rapidly developing callus, especially on the cut ends of the hypocotyl. The explants were scored for presence or absence of DsRed fluorescence on explants, regardless of size, intensity, and frequency on a single explant. The frequency of DsRed expressing sectors is an early indicator of transformation efficiency for a treatment.

The DsRed expression was markedly more intense in the constructs containing AtGRF5 or BnGRF5 than the DsRed control (Figure 15). The percent of explants with DsRed expressing shoots was significantly greater in explants that were transformed with either AtGRF5 or BnGRF5 than compared to explants transformed with the DsRed control (Table 7; Figure 16). Of the explants transformed with the DsRed control construct, 20.1% showed DsRed expressing shoots compared to 56.6% and 58.5% for AtGRF5 and GmGRF5, respectively. The data indicate that at 21 days after transformation, *B. napus* explants transformed with either AtGRF5 or BnGRF5 have nearly 3-fold more transgenic callus sectors than explants not transformed with either form of GRF5 (statistically significant α=0.05). Significant differences between researchers and experiments was not detected across the treatments (constructs).

**Table 7. Transformed callus was significantly increased on explants transformed with either AtGRF5 or BnGRF5 at 21 days after inoculation. The mean and the range in regeneration and DsRed fluorescing callus are shown. Constructs that were significantly different (at α=0.05) are followed with different letters; ¹= [(number of explants with DsRed callus/total number {n} of explants inoculated) × 100].**

| Construct | Exp. | Resear- chers | Replicates | Explants inoculated | Explants (%) with DsRed¹ | |
|---|---|---|---|---|---|---|
| | | | | | Mean | Range |
| Control | 5 | 3 | 20 | 1202 | 20.1 a | 13 - 28.7 |
| AtGRF5 | 4 | 3 | 15 | 822 | 56.6 b | 40.8 - 70.3 |
| BnGRF5 | 5 | 3 | 20 | 1132 | 58.5 b | 44.0-78.4 |

The invention is described by the following items:
1. A method for transforming a plant cell, comprising the steps
   (a1) introducing into a plant cell in parallel or sequentially
      i. at least one nucleotide sequence of interest; and
      ii. an expression cassette comprising a polynucleotide encoding a GRF5 polypeptide, mRNA encoding a GRF5 polypeptide, or GRF5 polypeptide(s); or
   (a2) introducing into a plant cell at least one nucleotide sequence of interest; and
      inducing in said plant cell in parallel or sequentially an enhanced expression level of an endogenous gene encoding a GRF5 polypeptide; and
   (b) optionally, cultivating the plant cell of (a1) or (a2) or a plant cell derived from the plant cell of (a1) or (a2) under conditions where in the plant cell the GRF5 polypeptide is expressed from the expression cassette, GRF5 polypeptide is translated from introduced mRNA, GRF5 polypeptide is enhanced expressed from the endogenous gene, or GRF5 polypeptide(s) are present.
2. A method for modifying the genome of a plant cell, comprising the steps
   (a1) introducing into a plant cell an expression cassette comprising a polynucleotide encoding a GRF5 polypeptide, mRNA encoding a GRF5 polypeptide, or GRF5 polypeptide(s); or
   (a2) inducing in a plant cell an enhanced expression level of an endogenous gene encoding a GRF5 polypeptide; and
   (b) cultivating the plant cell of (a1) or (a2) or a plant cell derived from the plant cell of (a1) or (a2) under conditions where in the plant cell the GRF5 polypeptide is expressed from the expression cassette, GRF5 polypeptide is translated from introduced mRNA, GRF5 polypeptide is enhanced expressed from the endogenous gene, or GRF5 polypeptide(s) are present;
   (c) modifying the genome of the plant cell of (b) by means of a double stranded DNA break (DSB) inducing enzyme which preferably recognize a predetermined site in the genome of said cell, and optionally by means of a repair nucleic acid molecule,
      wherein the modification of said genome at said predetermined site is selected from
      i. a replacement of at least one nucleotide;
      ii. a deletion of at least one nucleotide;
      iii. an insertion of at least one nucleotide; or
      iv. any combination of i. - iii.; and
   wherein step (c) is conducted simultaneously with step (a1)/(a2) and/or (b), before step (a1)/(a2), between step (a1)/(a2) and (b) or after step (b).
3. A method of producing a transgenic plant, comprising the steps
   (a) transforming a plant cell according to the method of item 1, and
   (b) regenerating from the plant cell of (a) or from a plant cell derived from the plant cell of (a) a plant comprising at least one cell which comprises the at least one nucleotide sequence of interest as transgene.
4. A method of producing a genetically modified plant, comprising the steps
   (a) modifying the genome of a plant cell according to the method of item 2, and
   (b) regenerating from the plant cell of (a) or from a plant cell derived from the plant cell of (a) a plant comprising in at least one cell the modification of the genome.
5. A method of producing a haploid plant embryo, comprising the steps
   (a1) introducing into an immature male gametophyte or a microspore an expression cassette comprising a polynucleotide encoding a GRF5 polypeptide, mRNA encoding a GRF5 polypeptide, or GRF5 polypeptide(s); or
   (a2) inducing in an immature male gametophyte or a microspore an enhanced expression level of an endogenous gene encoding a GRF5 polypeptide; and
   (c) cultivating the immature male gametophyte or the microspore of (a) under conditions where in the immature male gametophyte or the microspore the GRF5 polypeptide is expressed from the expression cassette, GRF5 polypeptide is translated from introduced mRNA, GRF5 polypeptide is enhanced expressed from the endogenous gene, or GRF5 polypeptide(s) are present; and
   (d) selecting haploid plant embryo derived from the immature male gametophyte or the microspore of step (b).
6. The method of any one of the items 1 to 5, wherein the GRF5 polypeptide comprises a PFAM domain PF08880 and a PFAM domain PF08879, preferably wherein the PFAM domain PF08880 finds a match of at least 90% coverage at or near the N-terminus of the GRF5 polypeptide and the PFAM domain PF08879 finds a match of at least 90% C-terminally located to the PFAM domain PF08880 in the GRF5 polypeptide.
7. The method of items 6, wherein both matching amino acid stretches are located in the N-terminal half of the GRF5 polypeptide, preferably the amino acid stretch matching PFAM domain PF08880 is located in the N-terminal quarter of the GRF5 polypeptide.
8. The method of any one of the items 1 to 7, wherein the GRF5 polypeptide comprises the motif [D]-[PL]-[E]-[P]-[G]-[R]-[C]-[R]-[R]-[T]-[D]-[G]-[K]-[K]-[W]-[R]-[C]-[SA]-[RK]-[ED]-[A]-[YH]-[P]-[D]-[S]-[K]-[Y]-[C]-[E]-[KR]-[H]-[M]-[H]-[R]-[G]-[RK]-[N]-[R] (SEQ ID NO: 177) with a maximum number of three mismatches, wherein preferably the motif consists of any of the amino acid sequences SEQ ID NO: 41 to SEQ ID NO: 104 or SEQ ID NO: 113 to SEQ ID NO: 176, and/or wherein preferably the motif contains a sub-region of amino acid stretch matching PFAM domain PF08879.
9. The method of any one of the items 1 to 8, wherein the GRF5 polypeptide comprises
   (i) an amino acid sequence comprising SEQ ID NO: 2, 4, 6, 8, 10, 12, 14, 16, 18, 20, 22, 24, 26, 28, 30, 32, 106, 108, 110, 112 or 209; or
   (ii) an amino acid sequence comprising a sequence being at least 70% identical to the amino acid of (i).
10. The method of any one of the items 1 to 9, wherein the polynucleotide encoding the GRF5 polypeptide comprises
   (i) a nucleotide sequence comprising SEQ ID NO: 1, 3, 5, 7, 9, 11, 13, 15, 17, 19, 21, 23, 25, 27, 29, 31, 105, 107, 109, 111, 207, 208 or 210;
   (ii) a nucleotide sequence comprising a sequence being at least 70% identical to the nucleotide sequence of (i);
   (iii) a nucleotide sequence encoding a polypeptide encoded by (i) or (ii) within the scope of the degeneracy of the genetic code;
   (iv) a nucleotide sequence complementary to a nucleotide sequence of (i), (ii) or (iii); or
   (v) a nucleotide sequence hybridizing with a nucleotide sequence of (iv) under stringent condition.
11. The method of any one of the items 1 to 10, wherein introducing into a plant cell the expression cassette comprising a polynucleotide encoding a GRF5 polypeptide results in a stable integration thereof into the genome of the plant cell, or wherein introducing into a plant cell the expression cassette comprising a polynucleotide encoding a GRF5 polypeptide, mRNA encoding GRF5 polypeptide, or GRF5 polypeptide(s) or inducing in a plant cell the enhanced expression level of an endogenous gene encoding a GRF5 polypeptide results in a transient occurrence of GRF5 polypeptide(s) in the plant cell or in a progeny cell thereof.
12. The method of any one of the items 1 to 11, wherein the polynucleotide encoding the GRF5 polypeptide is in operative linkage to at least one regulatory sequence suitable for expression of the GRF5 polypeptide in a plant cell.
13. The method of items 1 to 4, wherein the plant cell of step (a1) or (a2) is a cell of a somatic tissue, callus tissue, a meristematic tissue or an embryonic tissue, or a protoplast.
14. A plant obtained or obtainable by the method of item 3 or 4, or a progeny plant thereof.
15. A plant cell or a seed of the plant of item 14, wherein the plant cell or the seed comprises the at least one nucleotide sequence of interest as transgene or comprises the modification in the genome.

## Claims

1. Plant, obtained by a method comprising the steps
(a1) introducing into a plant cell in parallel or sequentially
i. at least one nucleotide sequence of interest; and
ii. an expression cassette comprising a polynucleotide encoding a GRF5 polypeptide, mRNA encoding a GRF5 polypeptide, or GRF5 polypeptide(s); or
(a2) introducing into a plant cell at least one nucleotide sequence of interest; and inducing in said plant cell in parallel or sequentially an enhanced expression level of an endogenous gene encoding a GRF5 polypeptide; and
(b) cultivating the plant cell of (a1) or (a2) or a plant cell derived from the plant cell of (a1) or (a2) under conditions where in the plant cell the GRF5 polypeptide is expressed from the expression cassette, GRF5 polypeptide is translated from introduced mRNA, GRF5 polypeptide is enhanced expressed from the endogenous gene, or GRF5 polypeptide(s) are present,
wherein the GRF5 polypeptide comprises a PFAM domain PF08880 and a PFAM domain PF08879; and
(c) regenerating a plant from the plant cell obtained in step (b) or from a plant cell derived from the plant cell obtained in step (b).

2. Plant, obtained by a method comprising the steps
(a1) introducing into a plant cell an expression cassette comprising a polynucleotide encoding a GRF5 polypeptide, mRNA encoding a GRF5 polypeptide, or GRF5 polypeptide(s); or
(a2) inducing in a plant cell an enhanced expression level of an endogenous gene encoding a GRF5 polypeptide;
wherein the GRF5 polypeptide comprises a PFAM domain PF08880 and a PFAM domain PF08879; and
(b) cultivating the plant cell of (a1) or (a2) or a plant cell derived from the plant cell of (a1) or (a2) under conditions where in the plant cell the GRF5 polypeptide is expressed from the expression cassette, GRF5 polypeptide is translated from introduced mRNA, GRF5 polypeptide is enhanced expressed from the endogenous gene, or GRF5 polypeptide(s) are present,
(c) modifying the genome of the plant cell of (b) by means of a double stranded DNA break (DSB) inducing enzyme, which preferably recognize a predetermined site in the genome of said cell, and optionally by means of a repair nucleic acid molecule;
wherein the modification of said genome at said predetermined site is selected from
i. a replacement of at least one nucleotide;
ii. a deletion of at least one nucleotide;
iii. an insertion of at least one nucleotide; or
iv. any combination of i. - iii.; and
wherein step (c) is conducted simultaneously with step (a1)/(a2) and/or (b), before step (a1)/(a2), between step (a1)/(a2) and (b) or after step (b), and
(d) regenerating a plant from the plant cell obtained in step (c) or from a plant cell derived from the plant cell obtained in step (c).

3. The plant according to claim 1 or 2, wherein the PFAM domain PF08880 finds a match of at least 90% coverage at or near the N-terminus of the GRF5 polypeptide and the PFAM domain PF08879 finds a match of at least 90% C-terminally located to the PFAM domain PF08880 in the GRF5 polypeptide.

4. The plant according to claim 3, wherein both matching amino acid stretches are located in the N-terminal half of the GRF5 polypeptide, preferably the amino acid stretch matching PFAM domain PF08880 is located in the N-terminal quarter of the GRF5 polypeptide.

5. The plant according to any one of claims 1-4, wherein the GRF5 polypeptide comprises the motif [D]-[PL]-[E]-[P]-[G]-[R]-[C]-[R]-[R]-[T]-[D]-[G]-[K]-[K]-[W]-[R]-[C]-[SA]-[RK]-[ED]-[A]-[YH]-[P]-[D]-[S]-[K]-[Y]-[C]-[E]-[KR]-[H]-[M]-[H]-[R]-[G]-[RK]-[N]-[R] (SEQ ID NO: 177) with a maximum number of three mismatches, wherein preferably the motif consists of any of the amino acid sequences SEQ ID NO: 41 to SEQ ID NO: 104 or SEQ ID NO: 113 to SEQ ID NO: 176, and/or wherein preferably the motif contains a sub-region of amino acid stretch matching PFAM domain PF08879.

6. The plant according to any one of claims 1-5, wherein the GRF5 polypeptide comprises
(iii) an amino acid sequence comprising SEQ ID NO: 2, 4, 6, 8, 10, 12, 14, 16, 18, 20, 22, 24, 26, 28, 30, 32, 106, 108, 110, 112 or 209; or
(iv) an amino acid sequence comprising a sequence being at least 70% identical to the amino acid of (i).

7. The plant according to any one of the claims 1-6, wherein the polynucleotide encoding the GRF5 polypeptide comprises
(vi) a nucleotide sequence comprising SEQ ID NO: 1, 3, 5, 7, 9, 11, 13, 15, 17, 19, 21, 23, 25, 27, 29, 31, 105, 107, 109, 111, 207, 208 or 210;
(vii) a nucleotide sequence comprising a sequence being at least 70% identical to the nucleotide sequence of (i);
(viii) a nucleotide sequence encoding a polypeptide encoded by (i) or (ii) within the scope of the degeneracy of the genetic code;
(ix) a nucleotide sequence complementary to a nucleotide sequence of (i), (ii) or (iii); or
(x) a nucleotide sequence hybridizing with a nucleotide sequence of (iv) under stringent condition.

8. The plant according to any one of claims 1-7, wherein introducing into a plant cell the expression cassette comprising a polynucleotide encoding a GRF5 polypeptide results in a stable integration thereof into the genome of the plant cell.

9. The plant according to any one of claims 1-8, which comprises the expression cassette comprising a polynucleotide encoding an exogenous GRF5 polypeptide.

10. The plant according to any one of claims 1-9, which comprises the at least one nucleotide sequence of interest as transgene or comprises the modification in the genome.

11. The plant according to any one of claims 1 - 10, which plant is of the genus selected from the genus selected from the group consisting of Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, or Helianthus,
preferably the plant is selected from the group consisting of Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp., including Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus and/or Allium tuberosum.

12. A combination of at least one nucleotide sequence of interest and an expression cassette comprising a polynucleotide encoding a GRF5 polypeptide,
wherein the GRF5 polypeptide comprises a PFAM domain PF08880 and a PFAM domain PF08879, and
wherein the nucleotide sequence of interest encodes at least one phenotypic trait selected from the group consisting of resistance/tolerance to biotic stress, resistance/tolerance to abiotic stress and resistance/tolerance against one or more herbicides, or wherein the at least one phenotypic trait is selected from the group consisting of the modification of yield increase, flowering time modification, seed colour modification, endosperm composition modification, nutritional content modification or metabolic engineering of a pathway of interest.

13. The combination according to claim 12, wherein the polynucleotide encoding the GRF5 polypeptide is in operative linkage to at least one regulatory sequence suitable for expression of the GRF5 polypeptide in a plant cell.

14. The combination according to claim 12 or 13, wherein the GRF5 polypeptide corresponds to the definition in any one of claims 4-7.

15. The combination according to any one of claims 12 to 14, wherein the polynucleotide encoding the GRF5 polypeptide comprises
(i) a nucleotide sequence comprising SEQ ID NO: 1, 3, 5, 7, 9, 11, 13, 15, 17, 19, 21, 23, 25, 27, 29, 31, 105, 107, 109, 111, 207, 208 or 210;
(ii) a nucleotide sequence comprising a sequence being at least 70% identical to the nucleotide sequence of (i);
(iii) a nucleotide sequence encoding a polypeptide encoded by (i) or (ii) within the scope of the degeneracy of the genetic code;
(iv) a nucleotide sequence complementary to a nucleotide sequence of (i), (ii) or (iii); or
(v) a nucleotide sequence hybridizing with a nucleotide sequence of (iv) under stringent condition.
